(19) Europäisches Patentamt
European Patent Office
Office européen des brevets

(11) Veröffentlichungsnummer: **0 132 770**
**B1**

(12) # EUROPÄISCHE PATENTSCHRIFT

(45) Veröffentlichungstag der Patentschrift: **16.09.87**

(51) Int. Cl.⁴: **C 07 K 7/40,** C 12 P 21/04, C 12 P 21/06, A 61 K 37/26

(21) Anmeldenummer: **84108442.9**

(22) Anmeldetag: **18.07.84**

(54) **Neue Insulin-Derivate, Verfahren zu deren Herstellung und deren Verwendung sowie pharmazeutische Mittel zur Behandlung des Diabetes mellitus.**

(30) Priorität: **22.07.83 DE 3326472**

(43) Veröffentlichungstag der Anmeldung:
**13.02.85 Patentblatt 85/7**

(45) Bekanntmachung des Hinweises auf die Patenterteilung:
**16.09.87 Patentblatt 87/38**

(84) Benannte Vertragsstaaten:
**AT BE CH DE FR GB IT LI LU NL SE**

(56) Entgegenhaltungen:
**EP - A - 0 017 938**
**EP - A - 0 045 187**
**EP - A - 0 089 007**
**EP - A - 0 092 280**
**US - A - 4 029 642**

(73) Patentinhaber: **HOECHST AKTIENGESELLSCHAFT, Postfach 80 03 20, D-6230 Frankfurt am Main 80 (DE)**

(72) Erfinder: **Grau, Ulrich, Dr., Zeil 17, D-6238 Hofheim an Taunus (DE)**

**EP 0 132 770 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der Therapie des Diabetes mellitus werden heute im allgemeinen Zubereitungen des blutzuckersenkenden Hormons Insulin parenteral verabreicht. Die spezielle Natur des Insulins und dessen Metabolismus bringen es mit sich, dass die Wirkungsdauer einer einfachen Lösung nur sehr kurz ist, dass also zur anhaltenden Blutzuckerkontrolle beim Diabetiker entweder eine Dauer-Infusion mit Dosiergeräten, mehrfache tägliche Injektionen oder eine verzögert wirksame Insulinzubereitung appliziert werden müssen. Als Verzögerungsprinzipien sind dabei von besonderer Bedeutung solche Zustandsformen des Insulins, die am Ort der Injektion schwerlöslich sind (z.B. kristallin oder amorph). Dazu zu rechnen sind beispielsweise Zink-Insulinkristalle oder Protamin-Insulin-Kristalle, die während ihrer langsamen Wiederauflösung über einen gewissen Zeitraum Insulin freisetzen.

Nun hat es sich bei der Therapie als äusserst hilfreich erwiesen, verschiedene Insulinpräparate zur Verfügung zu haben, die in ihrer Wirkungscharakteristik den Bedürfnissen des einzelnen Patienten möglichst nahe kommen. Im Zusammenhang mit nicht-optimaler Einstellung werden neben unmittelbaren Effekten wie Hyper- oder Hypoglykämien insbesondere die diabetischen Spätkomplikationen diskutiert, zu denen Retinopathie, Neuropathie, Nephropathie, Mikro- und Makrioangiopathie zählen.

Der Insulinmangel beim Diabetiker führt dazu, dass der Körper sein natürliches hormonelles Gleichgewicht nicht mehr erreichen kann.

Aufgabe der Erfindung ist die Bereitstellung eines Insulin-Derivates bzw. eines entsprechenden pharmazeutischen Mittels, durch das man sich dem natürlichen hormonellen Gleichgewicht in einem diabetischen Zustand besser annähern kann und wodurch dieses sich besser aufrechterhalten lässt als durch die Verabreichung von Insulin in den bisher üblichen Formen.

Diese Aufgabe wird nun erfindungsgemäss gelöst durch ein oder mehrere Insulin-Derivat(e), dessen (deren) B-Kette in der C-terminalen Region eine organische Gruppe basischen Charakters trägt bzw. durch ein pharmazeutisches Mittel, das dadurch gekennzeichnet ist, dass es dieses Insulin-Derivat als Wirkstoff enthält.

Insulin-Derivate, die am C-terminalen Ende der B-Kette die Reste Arg–OH oder Arg–Arg–OH tragen wurden schon beschrieben. Bekanntlich entstehen diese Derivate bei der enzymatischen Umwandlung von Proinsulin in Insulin in vivo als natürliche Zwischenprodukte und sind auch in kleinen Anteilen in Pankreasextrakten nachweisbar. Die genannten Reste werden normalerweise durch Trypsin und/oder Carboxypeptidase B oder Enzyme mit ähnlicher Spezifität unter Freisetzung des nativen Insulins abgespalten.

Die Erfindung betrifft Insulin-Derivate der Formel I,

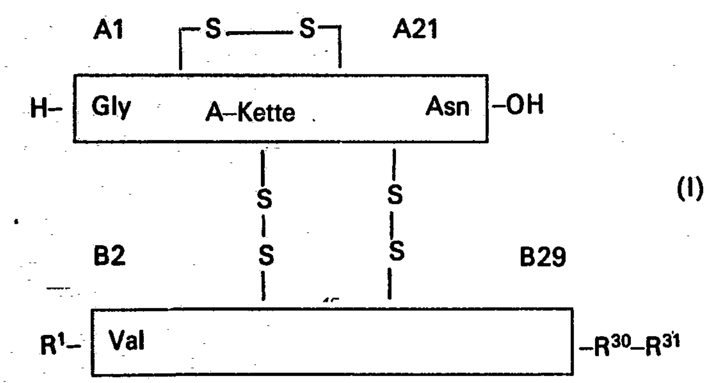

(I)

in welcher

$R^1$ H oder H–Phe bedeutet,

$R^{30}$ für den Rest einer neutralen, genetisch codierbaren L-Aminosäure steht und

$R^{31}$ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, wobei, falls $R^1$ für H–Phe steht, der C-Terminus $-R^{30}-R^{31}$ nicht $-Thr-(Arg)_m-OH$, $-Ala-(Arg)_m-OH$ oder $Ser-(Arg)_m-OH$ mit m=1 oder 2 bedeuten kann, mit der Massgabe, dass auch Verbindungen ausgenommen sind, in denen zugleich $R^1$ H–Phe, $R^{30}$ Thr und $R^{31}$ a) eine Aminosäure, b) ein Peptid oder c) ein Amid oder Ester von a) oder b) ist, gekennzeichnet durch einen isoelektrischen Punkt zwischen 5,8 und 8,5, sowie deren physiologisch verträglichen Salze.

Unter $R^{31}$ wird insbesondere ein Rest der Formel $-X_nS$ verstanden, in welcher

n = 0, 1, 2 oder 3 ist,

X = für gleiche oder verschiedene Reste natürlich vorkommender neutraler oder basischer L-Aminosäuren, vorzugsweise basischer L-Aminosäuren, insbesondere Arg, Lys, His oder Orn und/oder der diesen entsprechenden D-Aminosäuren steht und

S = OH oder eine physiologisch verträgliche, die Carboxygruppe blockierende Gruppe bedeutet, die falls n=0 ist, einen positiv geladenen oder protonierbaren basischen Rest trägt, oder, falls

$n>0$ ist, einen solchen Rest tragen kann und worin der C-Terminus

$-X-S$ auch für den Rest einer zum entsprechenden Alkohol reduzierten Aminosäure oder, im Falle $n=2$ oder 3, für den Homoserinlacton-Rest stehen kann.

Bevorzugt sind Insulin-Derivate der Formel I, in welcher $R^{30}$ für den Rest einer neutralen, genetisch kodierbaren L-Aminosäure steht,

a) $R^1$ H bedeutet und

$R^{31}$ a 1) eine physiologisch verträgliche, die Carboxygruppe blockierende Gruppe $S^B$, die einen positiv geladenen oder protonierbaren basischen Rest trägt, bedeutet,

a 2.1) für $X^N-S^B$ steht, worin $X^N$ den Rest einer natürlich vorkommenden neutralen L-Aminosäure oder deren D-Form bedeutet,

a 2.2) für $X^B-S$ steht, worin $X^B$ den Rest einer natürlich vorkommenden basischen L-Aminosäure oder deren D-Form und S OH oder eine die Carboxygruppe blockierende, gegebenenfalls einen positiv geladenen oder protonierbaren basischen Rest tragende Gruppe bedeutet,

a 2.3) für den Rest Y einer zum entsprechenden Alkohol reduzierten basischen Aminosäure $X^B$ steht,

a 3.1) für $-X_n-S$ steht, worin $n=2$ oder 3 ist, X die Reste $X^N$ und/oder $X^B$ bedeutet und S, falls alle Reste $X=X^N$ sind, nur $S^B$ bedeuten kann,

a 3.2) für $-X_n-Y$ steht, worin $n=1$ oder 2 ist,

a 3.3) für $-X^B-Z$, $-X^B-X^N-Z$, $-X^N-X^B-Z$ oder $-X^B-X^B-Z$ steht, worin $Z=Y$ ist oder den Homoserinlacton-Rest bedeutet oder

b) $R^1$ H-Phe bedeutet und $R^{30}$ Thr ist,

$R^{31}$ b 1) wie unter a 1) definiert ist,

b 2) wie unter a 2.3) definiert ist,

b 3) wie unter a 3.2) oder a 3.3) definiert ist, oder

c) $R^1$ H-Phe bedeuten und $R^{30}$ der Rest einer anderen neutralen, genetisch kodierbaren L-Aminosäure als Thr ist,

$R^{31}$ c 1) wie unter a 1) definiert ist,

c 2.1) wie unter a 2.1) definiert ist,

c 2.2) Lys-OH, D-Lys-OH, D-Arg-OH, Hyl-OH, D-Hyl-OH, Orn-OH, D-Orn-OH, Cit-OH, D-Cit-OH, His-OH oder D-His-OH bedeutet,

c 2.3) für $X^B-S'$ steht, worin S' die Bedeutung von S mit Ausnahme der von OH hat,

c 2.4) wie unter a 2.3) definiert ist,

c 3.1) für $-X-X'-OH$ oder $-X'-X-OH$ steht, worin X' wie unter c 2.2) definiert ist,

c 3.2) für $X_2-S'$ steht,

c 3.3) wie unter a 3.1) definiert ist, wobei $n=3$ ist,

c 3.4) wie unter a 3.2) oder a 3.3) definiert ist, oder dessen physiologisch verträglichen Salze.

Insulin-Derivat, die in Position B1 Phenylalanin tragen, sind besonders bevorzugt. Weiterhin sind solche bevorzugt, die in der Position B30 Ala, Thr oder Ser aufweisen.

Die A-Kette und die Kette (B2–29) der erfindungsgemässen Verbindungen weisen zweckmässigerweise die Sequenz des Rinder- oder Schweineinsulins, insbesondere aber die des Humaninsulins auf.

Die Aminosäurereste, X, $X^N$ und $X^B$ sowie die Reste Y und Z können unabhängig voneinander in der D- oder L-Konfiguration vorliegen. Bevorzugt ist jedoch die L-Konfiguration all dieser Reste.

Genetisch kodierbar sind die folgenden L-Aminosäuren: Gly, Ala, Ser, Thr, Val, Leu, Ile, Asp, Asn, Glu, Gln, Cys, Met, Arg, Lys, His, Tyr, Phe, Trp, Pro (neutrale Aminosäuren unterstrichen).

Unter einer neutralen, natürlich vorkommenden Aminosäure versteht man insbesondere Gly, Ala, Ser, Thr, Val, Leu, Ile, Asn, Gln, Cys, Met, Tyr, Phe, Pro oder Hyp. Unter einer basischen, natürlich vorkommenden Aminosäure versteht man insbesondere Arg, Lys, Hyl, Orn, Cit oder His.

Unter Gruppen, die gegebenenfalls eine freie Carboxyfunktion am C-terminalen Ende der B-Kette in den erfindungsgemässen Verbindungen blockieren, versteht man vor allem Ester- und Amidgruppen, vorzugsweise ($C_1$ bis $C_6$)-Alkoxy, ($C_3$ bis $C_6$)-Cycloalkyloxy, $NH_2$, ($C_1$ bis $C_6$)-Alkylamino, Di-($C_1$ bis $C_6$)-alkylamino oder basische Gruppen, wie Amino-($C_2$ bis $C_6$)-alkoxy, ($C_1$ bis $C_4$)-Alkylamino-($C_2$ bis $C_6$)-alkoxy, Di-($C_1$ bis $C_4$)-alkylamino-($C_2$ bis $C_6$)-alkoxy, Tri-($C_1$ bis $C_4$)ammonio-($C_2$ bis $C_6$)-alkoxy, Amino ($C_2$ bis $C_6$)-alkylamino, [($C_1$ bis $C_4$)-Alkylamino] -($C_2$ bis $C_6$)alkylamino, [Di-($C_1$–$C_4$)-alkylamino]-($C_2$–$C_6$)-alkylamino oder [Tri($C_1$ bis $C_4$)-alkylamino]-($C_2$ bis $C_6$)-alkylamino, insbesondere $-O-[CH_2]_p-NR_2$, $-O-[CH_2]_p-N^{\oplus}R_3$, $-NH-[CH_2]_p-NR_2$ oder $-NH-[CH_2]_p-N^{\oplus}R_3$, worin $p=2$ bis 6 ist und R gleich oder verschieden ist und für Wasserstoff oder ($C_1$ bis $C_4$)-Alkyl steht.

In der Reihe der erfindungsgemässen Insulin-Derivate seien beispielsweise die nachstehenden Verbindungen erwähnt, ohne die Erfindung auf diese zu beschränken:

Des-$Phe^{B1}$-Schweineinsulin-$Arg^{B31}$-OH
Des-$Phe^{B1}$-Humaninsulin-$Arg^{B31}$-OH
Des-$Phe^{B1}$-Schweineinsulin-$Arg^{B31}$-$Arg^{B32}$-OH
Des-$Phe^{B1}$-Humaninsulin-$Arg^{B31}$-$Arg^{B32}$-OH
Schweineinsulin-$Arg^{B31}$-$OCH_3$
Humaninsulin-$Arg^{B31}$-$OCH_3$
Rinderinsulin-$Arg^{B31}$-$OCH_3$
Schweineinsulin-$Arg^{B31}$-$Arg^{B32}$-$OCH_3$
Humaninsulin-$Arg^{B31}$-$Arg^{B32}$-$OCH_3$
Des-$Thr^{B30}$-Humaninsulin-$Val^{B30}$-$Arg^{B31}$-OH
Des-$Thr^{B30}$-Humaninsulin-$Val^{B30}$-$Ala^{B31}$-$Arg^{B32}$-OH

Humaninsulin-$Lys^{B31}$-OH
Humaninsulin-D-$Arg^{B31}$-OH
Humaninsulin-D-$Arg^{B31}$-$Arg^{B32}$-OH
Humaninsulin-$Arg^{B31}$-D-$Arg^{B32}$-OH
Humaninsulin-$Lys^{B31}$-$Arg^{B32}$-OH
Humaninsulin-$Arg^{B31}$-$Lys^{B32}$-OH
Humaninsulin-$Argininol^{B31}$
Humaninsulin-$Val^{B31}$-$Arg^{B32}$-OH
Humaninsulin-$Val^{B31}$-$Arg^{B32}$-$Arg^{B33}$-OH
Humaninsulin-$Arg^{B31}$-$Argininol^{B32}$
Humaninsulin-$Lys^{B31}$-$Arg^{B32}$-$Arg^{B33}$-OH

Humaninsulin-$Arg^{B31}$-NH 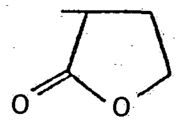

Humaninsulin-$Arg^{B31}$-$Arg^{B32}$-NH 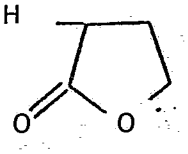

Humaninsulin-$Arg^{B31}$-$NH_2$
Humaninsulin-$Arg^{B31}$-$Arg^{B32}$-$NH_2$
Humaninsulin-$Orn^{B31}$-OH
Humaninsulin-$Leu^{B31}$-$Cit^{B32}$-OH
Humaninsulin-(B30)-$OCH_2CH_2$–$NH_2$
Humaninsulin-(B30)-NH-$CH_2CH_2$-$NH_2$
Humaninsulin-$Arg^{B31}$-O-$CH_2$-$CH_2$-$NH_2$
Humaninsulin-$Arg^{B31}$-$CH_2$-$CH_2$-$N(CH_3)_2$
Humaninsulin-(B30)-O-$CH_2$-$CH_2$-$N^{\oplus}(CH_3)_3$
Humaninsulin-(B30)-NH-$CH_2$-$CH_2$-$N^{\oplus}(CH_3)_3$
Humaninsulin-$Leu^{B31}$-O-$CH_2$-$CH_2$-$CH_2$-$N^{\oplus}(C_2H_5)_3$
Humaninsulin-$Trp^{B31}$-$Trp^{B32}$-$Trp^{B33}$-$NH(CH_2)_6$-$N^{\oplus}[(CH_2)_3CH_3]_3$

Die Erfindung betrifft auch ein Verfahren zur Herstellung von Insulin-Derivaten der Formel I das dadurch gekennzeichnet ist, dass man

a) ein Des-Octapeptid (B23–30)-Insulin der Formel II,

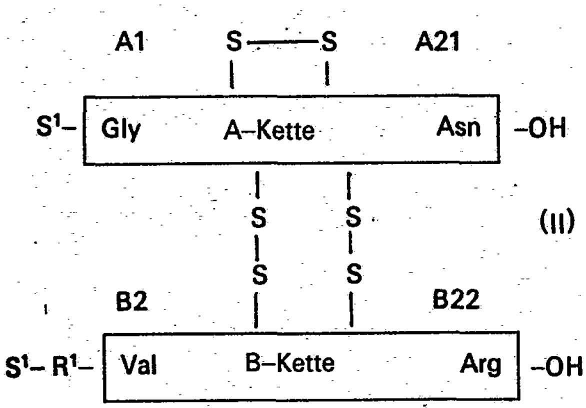

(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder den Methylsulfonylethyloxycarbonyl-(Msc)-rest bedeuten, kondensiert mit einem Peptid der Formel III

H–Gly–Phe–Phe–Tyr)$S^2$)–Thr($S^2$)–Pro-Lys($S^3$)-$R^{30}$-$R^{31}$, (III)

in welcher $R^{30}$ und $R^{31}$ die oben definierten Bedeutungen haben, $S^2$ für Wasserstoff, Bzl oder $Bu^t$ und $S^3$ für eine Urethanschutzgruppe, wie Boc, Moc, Fmoc oder Z stehen, wobei in den Resten $R^{30}$ und $R^{31}$ vorhandene frei COOH–, OH–, SH–, $NH_2$–, Guanidino- und/oder Imidazol-Gruppen, falls erforderlich, in an sich bekannter Weise geschützt vorliegen, und gegebenenfalls vorhandene Schutzgruppen in an sich bekannter Weise abspaltet,

b) ein Des-B30-Insulin der Formel I, in welcher $R^1$ für H oder H-Phe und der C-Terminus $R^{30}$–$R^{31}$ zusammen für OH stehen, in Gegenwart von Trypsin oder einer trypsinähnlichen Endopeptidase umsetzt mit einer Verbindung der Formel IV

H–$R^{30}$–$R^{31}$ (IV)

in der $R^{30}$ und $R^{31}$ die oben definierten Bedeutungen haben und worin vorhandene freie COOH–, OH–, SH–, ω–$NH_2$–, Guanidino- und/oder Imidazol-Funktionen, falls erforderlich, in an sich bekannter Weise geschützt vorliegen und anschliessend gegebenenfalls vorhandene Schutzgruppen in an sich bekannter Weise abspaltet, oder

c) zur Herstellung eines Insulin-Derivates mit L-konfigurierten Aminosäureresten in $R^{31}$ ein Proinsulin, Proinsulinanaloges oder Präproinsulinanaloges oder ein Intermediat dieser Verbindungen chemisch und/oder enzymatisch spaltet.

Bei der Verfahrensvariante a) setzt man beispielsweise das $N^{\alpha A1}$, $N^{\alpha B1}$-Bis-Boc-Derivat eines Des-Octapeptid-(B23–30) Insulins analog der im US-Patent 4 029 642 beschriebenen Verfahrensweise direkt mit einem Äquivalent einer Verbindung der Formel III um, wobei als Kondensationsmittel Dicyclohexylcarbodiimid im geringen Unterschuss in Anwesenheit von 1-Hydroxybenzotriazol dient.

Da bei dieser Verfahrensvariante gewöhnlich kein Schutz der Carboxylgruppen erforderlich ist, entfällt normalerweise auch die Schädigung des Insulin-Derivats sowohl bei der Veresterung als auch bei der alkalischen Verseifung. Nicht umgesetztes Des-Octapeptid und ein Peptid, das durch Kondensation von IV an $Asp^{A21}$–OH entsteht, sind auf Grund der unterschiedlichen Molekülgrösse und Ladungszahl leicht durch Verteilungschromatographie an $Sephadex^R$-LH 20 oder durch Gelchromatographie an Sephadex®-G 75 oder G 50 superfine abtrennbar.

Zur Abspaltung der tert-Butylschutzgruppen muss das Reaktionsprodukt nur mit Trifluoressigsäure während 30–60 Minuten bei Raumtemperatur behandelt werden. Diese Reaktion schädigt das Insulin-Derivat nicht. Wählt man als N-Schutzgruppe den Methylsulfonylethyl-oxy-carbonylrest, so ist zur Abspaltung durch β-Eliminierung eine Alkalibehandlung notwendig. Die Reaktionsbedingungen sind so (z.B. 0,1 N NaOH, 0 °C, 5 sek), dass das Insulin-Derivat nicht geschädigt wird. Das als Ausgangsprodukt verwendete $N^{\alpha A1}$, $N^{\alpha B1}$-Bis-Boc-Des-$B_{23-30}$-Octapeptid-Insulin vom Schwein stellt man beispielsweise auf folgendem Wege her:

Schweineinsulin wird in einer Mischung aus Dimethylformamid, Dimethylsulfoxid und Wasser in Anwesenheit von N-Ethylmorpholin mit überschüssigem tert.-Butyloxycarbonyl-N-hydroxysuccinimidester umgesetzt. Dabei entsteht das zu erwartende $N^{\alpha A1}$, $N^{\alpha B1}$, $N^{\varepsilon B29}$-Tris-Boc-Insulin.

Man gibt nun zu der Lösung dieser Verbindung in Dimethylformamid und Tris-Puffer (pH 7,5) Trypsin in kleinen Portionen so lange zu, bis in der Elektrophorese kein Ausgangsprodukt mehr zu erkennen ist. Das $N^{\alpha A1}$, $N^{\alpha B1}$-Bis-Boc-Des-$B_{23-30}$-Octapeptid-Insulin wird durch Verteilungschromatographie an Sephadex® LH 20 gereinigt.

Diese Verbindung wird nun mit einem Mol des Peptids der Formel III, das in an sich bekannter Weise nach den Methoden der Peptidchemie hergestellt wird, 1–2 Mol 1-Hydroxybenzotriazol und etwa 0,9 Mol Dicyclohexylcarbodiimid in Dimethylformamid bei etwa pH 7–8 zur Reaktion gebracht (vgl. Chem. Ber. 103 (1970), Seite 788).

Das Rohprodukt wird durch Verteilungschromatographie gereinigt und durch Behandeln mit Trifluoressigsäure/Anisol bei Raumtemperatur von den Schutzgruppen befreit. Nach Fällung mit Ether, isoelektrischer Fällung aus Wasser und Chromatographie an Sephadex®-G 75 oder G 50 superfine ist die Verbindung elektrophoretisch rein und kann in bekannter Weise zur Kristallisation gebracht werden. Das so gewonnene Insulin-Derivat ist biologisch voll aktiv.

Des-$Phe^{B1}$-Insuline als Ausgangsverbindungen für die erfindungsgemässen Verfahren sind beispielsweise aus der DE-PS 20 05 658 oder aus der EP-A-46 979 bekannt.

Die bei der Verfahrensvariante b) als Ausgangsverbindungen verwendeten Des-B30-Insuline sind beispielsweise aus der EP-A-46 979 oder Hoppe-Seyler's Z. Physiol. Chem. 359 (1978) 799 bekannt. Das bei Variante b) verwendete Ausgangsmaterial der Formel IV wird in an sich bekannter Weise nach den Methoden der Peptidchemie hergestellt. Brauchbare Schutzgruppen für IV sind detailiert beschrieben bei M. Bodanzyky et al., Peptide Synthesis, Ind. Ed. 1976, Wiley & Sons.

Das Des-B30-Insulin und die Verbindung der Formel IV werden in Analogie zu der im US-Patent 4 320 196 beschriebenen Verfahrensweise in Gegenwart von Trypsin oder einer trypsinähnlichen Endopeptidase in einem organisch-wässrigen Lösemittel-System bei pH 5–9 und bei einer Temperatur von 20 bis 40 °C miteinander kondensiert. Das erhaltene Insulin-Derivat kann nach den gebräuchlichen Methoden der Peptidchemie isoliert werden.

Durch gentechnologische Methoden ist inzwischen humanes oder Primaten-Proinsulin als Ausgangsmaterial für die Verfahrensvariante c) zugänglich. Die Derivate Arg(B31) und Di-Arg(B31–32) sind daraus durch einfache Verdauung mit Trypsin oder Trypsin-ähnlichen Enzymen zugänglich. Daneben lassen sich aber auch relativ einfach Plasmide konstruieren, die durch Spaltung entsprechender Präproinsulinderivate zu neuen Insulinderivaten führen, weil sie an Stelle der natürlich an B31 oder B32 befindlichen Arginine für andere neutrale oder basische Aminosäuren codieren.

Die Herstellung von Proinsulin unter Anwendung der Rekombinations-DNA-Methodologie erfordert die Bildung einer DNA-Sequenz, die für die Aminosäuresequenz eines Proinsulins codiert, was sich entweder durch Isolierung, Konstruktion oder eine Kombination aus beidem erreichen lässt. Die Proinsulin-DNA wird dann in Lesephase in einen geeigneten Clonierungs- und Expressionsträger eingesetzt. Der Träger dient zur Transformierung eines geeigneten Mikroorganismus, und der hierbei erhaltene transformierte Mikroorganismus wird dann Fermentationsbedingungen unterzogen, die zur Bildung weiterer Kopien des proinsulingenhaltigen Vektors und zur Expression von Proinsulin, eines Proinsulinderivats oder eines Proinsulinvorläufers (bzw. eines Präproinsulinderivats) führen.

Handelt es sich beim Expressionsprodukt um einen Proinsulinvorläufer, dann enthält ein solches Produkt im allgemeinen die Proinsulinaminosäuresequenz, die an ihrer endständigen Aminogruppe an ein Bruchstück eines Proteins gebunden ist, das normalerweise durch die Gensequenz ausgedrückt wird, in welcher das Proinsulin oder Proinsulinderivat eingesetzt worden ist. Die Proinsulinaminosäuresequenz ist an das Proteinbruchstück über eine spezifisch spaltbare Stelle gebunden, bei der es sich beispielsweise um Methionin handelt. Die erhaltene Proinsulinaminosäuresequenz wird vom verschmolzenen Genprodukt, beispielsweise wie in der DE-A-32 32 036 beschrieben, abgespalten und das Proinsulin nach Reinigung isoliert.

Die enzymatische Spaltung des auf diese Weise erhaltenen Proinsulins oder Proinsulinderivats erfolgt in Analogie zu der in Excerpta Medica International Congress Series No. 231, Seite 292 ff. oder der in der deutschen Patentanmeldung P 32 09 184 (HOE 82/F 047) beschriebenen Verfahrensweise.

Mit Hilfe der beschriebenen semisynthetischen Verfahren sind zusätzlich zu den bekannten Arginin (B30)- und Diarginin (B31–32)-Derivaten sowie jenen durch gentechnologische Methoden zugänglichen Derivaten, die in $R^{31}$ natürliche L-Aminosäuren tragen, eine Reihe von neuen Insulin-Derivaten zugänglich, die als Charakteristikum eine oder mehrere basische Gruppe(n) und/oder die Abwesenheit der freien Carboxylgruppe aufweisen, so dass die Nettoladung des Moleküls um mindestens eine positive Ladung gegenüber nativem Insulin oder gegenüber Des-$Phe^{B1}$-Insulin zunimmt.

Dazu gehören z.B. Derivate, die an Position B31 statt der natürlichen Aminosäuren L-Lysin, L-Histidin oder L-Arginin deren D-Enantiomere oder gängige D- oder L-Aminosäureanaloge, die in der Seitenkette eine basische Gruppierung (z.B. Ornithin, Hydroxylysin tragen, aufweisen. Statt einer Aminosäure kann an Stelle der Position B31 beispielsweise die Cholinestergruppe treten, wodurch netto zwei Positivladungen gewonnen werden. Die Aminosäure oder das Aminosäureanalogon an Position B31 kann ein freies Carboxylende aufweisen oder mit einfachen Alkoholen (z.b. Methanol, Ethanol) verestert bzw. mit einfachen Stickstoffbasen (z.B. Ammoniak,

mono-, di- Methylamin) amidiert sein; sie kann daneben z.B. mit Cholin verestert sein. An Position B32 kann beispielsweise eine neutrale oder auch eine weitere natürliche basische Aminosäure oder eines der oben beschriebenen Aminosäurederivate folgen; in analoger Weise kann deren Carboxylgruppe frei oder verestert bzw. amidiert sein. Es kann beispielsweise auch hier die Cholinestergruppe bzw. noch eine weitere neutrale oder basische Aminosäure bzw. ein Aminosäureanalogon folgen.

Allen diesen Insulinderivaten ist gemeinsam, dass die zusätzliche(n), an der Oberfläche des Moleküls befindliche(n) positive(n) Ladung(en) dem Molekül einen in den Neutralbereich hinein verschobenen isoelektrischen Punkt verleihen. Je nach Derivat werden isoelektrische Punkte von 5,8 bis 8,5, insbesondere 6,2 bis 8,2 in der isoelektrischen Fokussierung gemessen. Damit sind die Derivate im Neutralbereich weniger löslich als natives Insulin oder Proinsulin, die ihren isoelektrischen Punkt und damit den Bereich maximaler Unlöslichkeit bei pH=5,4 haben, während sie im Neutralbereich normalerweise gelöst vorliegen.

Die Löslichkeitseigenschaften von Insulin und Proinsulin lassen sich im Bereich oberhalb des isoelektrischen Punktes, d.h. im therapeutisch besonders interessanten Neutralbereich, durch Zugabe von Zinkionen beeinflussen. Zink wirkt dabei als Depotprinzip in der Weise, dass es den hexameren Zustand des Insulins sowie dessen Kristallisierungstendenz stabilisiert. Im subkutanen Gewebe lösen sich diese Aggregate wieder.

Ein weiteres geläufiges Depotprinzip ist die Kristallisation des Insulins oder Proinsulins als Komplex mit einem basischen Protein, z.B. Globin oder Protamin.

Bei der Anwendung des Proinsulins in Lösung oder in Verbindung mit einem der beschriebenen Depotprinzipien bedarf es eines weiteren proteolytischen Abbaus, um natives, voll wirksames Insulin freizusetzen. Intaktes Proinsulin hat nur etwa 1/8 der biologischen Wirksamkeit des Insulins, weil, so die Vorstellung, ein Teil der biologisch aktiven Oberflächenregion, die Rezeptor-Bindungsregion, durch das im Proinsulin vorhandene C-Peptid maskiert ist. Als Proinsulin kommt allerdings für die Diabetestherapie nur homologes, also nur solches mit menschlicher Sequenz, in Frage (vgl. z.B. DE-A1-32 32 036). Heterologes Proinsulin besitzt eine signifikante Immunogenität. Es ist in diesem Zusammenhang bemerkenswert, dass auch humane Proinsuline Variationen im C-Peptidteil aufweisen können.

Schweineinsulin-Arg$^{B31}$OH und das entsprechende Diarginin-Derivat weisen den Untersuchungen von Chance, Excerpta Medica International Congress Series No. 231, Seite 292, 293 zufolge nur 62% bzw. 66% der Aktivität des nativen Schweineinsulins auf.

Überraschenderweise wurde nun gefunden, dass Insulin-Arg$^{B31}$–OH, Insulin-Arg$^{B31}$-Arg$^{B32}$–OH und andere Insulin-Derivate, deren B-Kette C-terminal eine organische Gruppe basischen Charakters trägt, im Unterschied zum Proinsulin eine etwa gleich hohe biologische Aktivität wie natives Insulin aufweisen.

Die Erfindung betrifft daher Arzneimittel zur Behandlung des Diabetes mellitus aus einem pharmazeutisch unbedenklichen Träger und einem Wirkstoff, dadurch gekennzeichnet, dass es als Wirkstoff ein Insulin-Derivat mit einem isoelektrischen Punkt zwischen 5,8 und 8,5 und der Formel I, in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, oder eines seiner physiologisch verträglichen Salze enthält.

Die erfindungsgemässen Arzneimittel stellen weiterhin vollkommen neue Verzögerungsprinzipien dar, die ohne Depot-Hilfsstoffe, wie Zink oder Protaminsulfat, zur Wirkung gebracht werden können. Die Depotwirkung geht auf ein inhärentes, proteinchemisch bedingtes, physikalisches Prinzip, die Schwerlöslichkeit des Insulin-Derivats an dessen isoelektrischem Punkt, zurück. Seine Wiederauflösung unter physiologischen Bedingungen wird möglicherweise durch Abspaltung der zusätzlichen basischen Gruppen erreicht, die je nach Derivat durch tryptische oder trypsinähnliche und/oder Carboxypeptidase B oder der Carboxypeptidase B ähnliche und/oder Esterase-Aktivität zustande kommt. Die jeweils abgespaltenen Gruppen sind entweder rein physiologische Metaboliten, wie Aminosäuren, Ornithin oder Cholin, oder aber leicht metabolisierbare, physiologisch unbedenkliche Substanzen.

Die Insulin-Derivate als Wirkstoffe dieser neuen Arzneimittel sind auch im Unterschied zu in der Literatur beschriebenen Intermediaten, die noch Teile des heterologen C-Peptids enthalten, nicht stärker immunogen als das entsprechende Insulin selbst.

Die obengenannten zu niedrigen Aktivitätswerte von Chance haben möglicherweise ihre Ursache in einer ungenügenden Reinheit der untersuchten Fraktionen oder in einem systematischen Messfehler. Ihre Verwendbarkeit als Arzneimittelwirkstoffe wurden jedenfalls bisher (und vielleicht daher) noch nicht erkannt.

Die erfindungsgemässen Mittel enthalten als Wirkstoff eines oder mehrere der neuen Insulin-Derivate der Formel I oder Insulin-Arg$^{B31}$–OH oder Insulin-Arg$^{B31}$-Arg$^{B32}$–OH.

Sie weisen vorzugsweise einen pH-Wert zwischen 2,5 und 8,5 auf, enthalten ein geeignetes Isotoniemittel, ein geeignetes Konservierungsmittel und gegebenenfalls einen geeigneten Puffer für einen pH-Bereich zwischen 5,0 und 8,5.

Eine typische Anwendungsform der beschriebenen Derivate stellen Präparationen dar, die unterhalb des isoelektrischen Punktes als Lösungen in einem physiologisch unbedenklichen Träger

vorliegen. Dabei kann der pH der Lösung typischerweise pH=5,0 betragen, liegt also deutlich höher als der von sauren Altinsulinen (typischerweise pH=3,0). Eine neutralere Injektionslösung bietet u.U. deutliche Vorteile in Bezug auf deren Verträglichkeit.

Eine weitere typische Anwendungsform sind Suspensionen aus amorphen oder kristallinen Niederschlägen der beschriebenen Derivate in einem physiologisch unbedenklichen Träger von etwa neutralem pH.

Es ist aber auch möglich, die in den Derivaten inhärente Schwerlöslichkeit im physiologischen pH-Bereich durch zusätzliche Depotprinzipien, wie etwa durch Zugabe von Zink oder Protaminsulfat, zu verstärken. Die zugesetzte Zinkmenge kann dabei bis zu 100 µg $Zn^{2+}$/100 Insulineinheiten, typischerweise etwa 50 µg $Zn^{2+}$/100 Insulineinheiten, betragen. Die Protaminmenge kann zwischen 0,28 mg und 0,6 mg pro 100 Einheiten betragen (bezogen auf Protaminsulfat). Auf diese Weise sind besonders lang wirksame Präparationen herstellbar für die es in Zukunft breitere Anwendung als bisher geben wird, nachdem gerade eine Basalmenge an Insulin therapeutisch vorteilhaft erscheint. Dies ist schon jetzt eine Erkenntnis aus der Therapie mit Insulindosiergeräten.

Als physiologisch unbedenkliches und mit dem Insulinderivat verträgliches Trägermedium eignet sich eine sterile wässrige Lösung, die zu Blut in der üblichen Weise, z.B. durch Glycerin, Kochsalz, Glucose, isotonisch gemacht wird und daneben noch eines der gebräuchlichen Konservierungsmittel z.B. Phenol, m-Kresol oder p-Hydroxybenzoesäureester, enthält. Das Trägermedium kann zusätzlich eine Puffersubstanz, z.B. Natriumacetat, Natriumcitrat, Natriumphosphat, enthalten. Zur Einstellung des pH werden verdünnte Säuren (typischerweise HCl) bzw. Laugen (typischerweise NaOH) verwendet.

Die Insulin-Derivate können in den erfindungsgemässen Mitteln auch als Alkali- oder als Ammoniumsalze eingesetzt werden. Ein beliebiger Anteil eines oder mehrerer Insulin-Derivate der Formel I oder ein Insulin-Derivat der Formel I kann in einer Mischung weiterer dieser Insulin-Derivate unabhängig voneinander jeweils in gelöster, amorpher und/oder kristalliner Form vorliegen.

Es ist manchmal vorteilhaft, der erfindungsgemässen Zubereitung eine geeignete Menge eines geeigneten Stabilisators zuzusetzen, der die Präzipitation von Protein bei thermisch-mechanischer Belastung bei Kontakt mit verschiedenen Materialien verhindert. Solche Stabilisatoren sind beispielsweise aus der EP-A-18609, der DE-A-32 40 177 oder aus der WO-83/00288 bekannt.

Bei den erfindungsgemässen Mitteln, die auch eines der bekannten Verzögerungsprinzipien, wie etwa Protaminsulfat, Globin oder Zink in geeigneten Mengen enthalten können, kann ein solches Verzögerungsprinzip in Kombination mit dem gesamten Wirkstoffanteil oder mit Teilen davon oder einem oder mehrerern Insulin-Derivaten der Formel I in einer Mischung angewandt werden. Ein Mittel kann verschiedene Insulin-Derivate der Formel I in Kombination mit mehreren verschiedenen verzögernd wirkenden Hilfsstoffen enthalten.

Mit den erfindungsgemässen therapeutischen Mitteln sind also offenkundig vielfältige und sehr fein abstimmbare Wirkungscharakteristiken erzielbar; damit sollten nach den eingangs gemachten Bemerkungen Fortschritte insbesondere im Hinblick auf diabetische Spätkomplikationen verbunden sein.

Zur weiteren Erläuterung der Erfindung sollen die folgenden Beispiele dienen:

Herstellungsbeispiel 1:
Humaninsulin-(B30)–O–$CH_2$–$CH_2$–$N^{\oplus}(CH_3)_3$

5 g Schweineinsulin werden in 45 ml Dimethylformamid, 25 ml Dimethylsulfoxid, 0,5 ml N-Ethylmorpholin und 2,5 ml Wasser gelöst. Unter Rühren gibt man bei Raumtemperatur 1,5 g tert-Butyloxycarbonyl-N-hydroxysuccinimid zu und lässt 6 Stunden reagieren. Dann wird durch Zugabe eines Tropfens Eisessig gestoppt, das Produkt mit Ether gefällt und abfiltriert. Der Rückstand wird in 360 ml Dimethylformamid gelöst und mit 320 ml Tris-Puffer (0,05 M, 0,01 M an $CaCl_2$, pH 7,5) verdünnt. Bei 36°C werden im Abstand von je 1 Stunde Portionen von 20 mg Trypsin zugegeben.

Nach insgesamt 12 Zugaben stellt man mit Essigsäure auf pH 4,5 und dampft die Lösung ein. Die anschliessende Reinigung des Materials an einer Sephadex®-LH 20-Säule (8×200 cm) mittels Verteilungschromatographie im System n-Butanol-Eisessig-Wasser (2:1:10) ergibt 3,25 g $N^{\alpha A1}$, $N^{\alpha B1}$-Bis-Boc-Des-$R_{23\text{-}30}$-Octapeptid-Insulin (Schwein), das in der sauren und basischen Elektrophorese kein Ausgangsmaterial mehr zeigt. Die Aminosäureanalyse der Substanz ist korrekt. Nach einer probeweisen Abspaltung der HOE-Gruppen ist keine Insulinaktivität mehr zu finden. Dieses Material (3,25 g) wird in 30 ml Dimethylformamid zusammen mit 100 mg 1-Hydroxybenzotriazol, 750 mg HCl·Gly-Phe-Phe-Tyr($Bu^t$)-Thr-Pro-Lys(Boc)-Thr($Bu^t$)-$OCH_2CH_2$-$N^{\oplus}$ $(CH_3)_3$·HCl und 0,5 ml N-Ethylmorpholin gelöst. Dann gibt man bei Raumtemperatur 120 mg Dicyclohexylcarbodiimid zu und rührt die Reaktion 24 Stunden. Der abgeschiedene Dicyclohexylharnstoff wird abfiltriert, das Produkt durch Etherzugabe gefällt.

Den Niederschlag filtriert man ab, wäscht mit Ether und trocknet. Die Sutanz wird durch Verteilungschromatographie an Sephadex®-LH 20 im obigen System vorgereinigt. 2,6 g Material aus dem Hauptpeak isoliert man durch Fällung mit Aceton/Ether. Das getrocknete, noch ungeschützte Derivat lässt man mit einem Gemisch aus 5 ml Trifluoressigsäure und 1 ml Anisol 60 Minuten bei Raumtemperatur reagieren. Aus der mit Eis gekühlten Lösung fällt man anschliessend die Rohsubstanz durch Zugabe von Ether. Den getrockneten Niederschlag löst man in Wasser, präzipitiert mit wässrigem Ammoniak und zentrifugiert. Die Reinigung des Produktes erfolgt in 10%iger Essigsäure über Sephadex[R]-G 50 superfine oder G 75. Aus den Fraktionen des gewünschten Peaks kann man das Humaninsulin-(B30)–$OCH_2CH_2N^{\oplus}(CH_3)_3$ durch Gefriertrocknung isolieren (Ausbeute nach Kristallisation: 1,2 g). Das so gewonnene Insulin-Derivat zeigt im biologischen Test eine dem Humaninsulin äquivalente Wirksamkeit.

Die Herstellung des Oktapeptids der Formel III erfolgt gemäss folgendem Kondensationsschema nach üblichen Peptid-Kondensationsmethoden:

Syntheseschema für das Octapeptid der Formel III

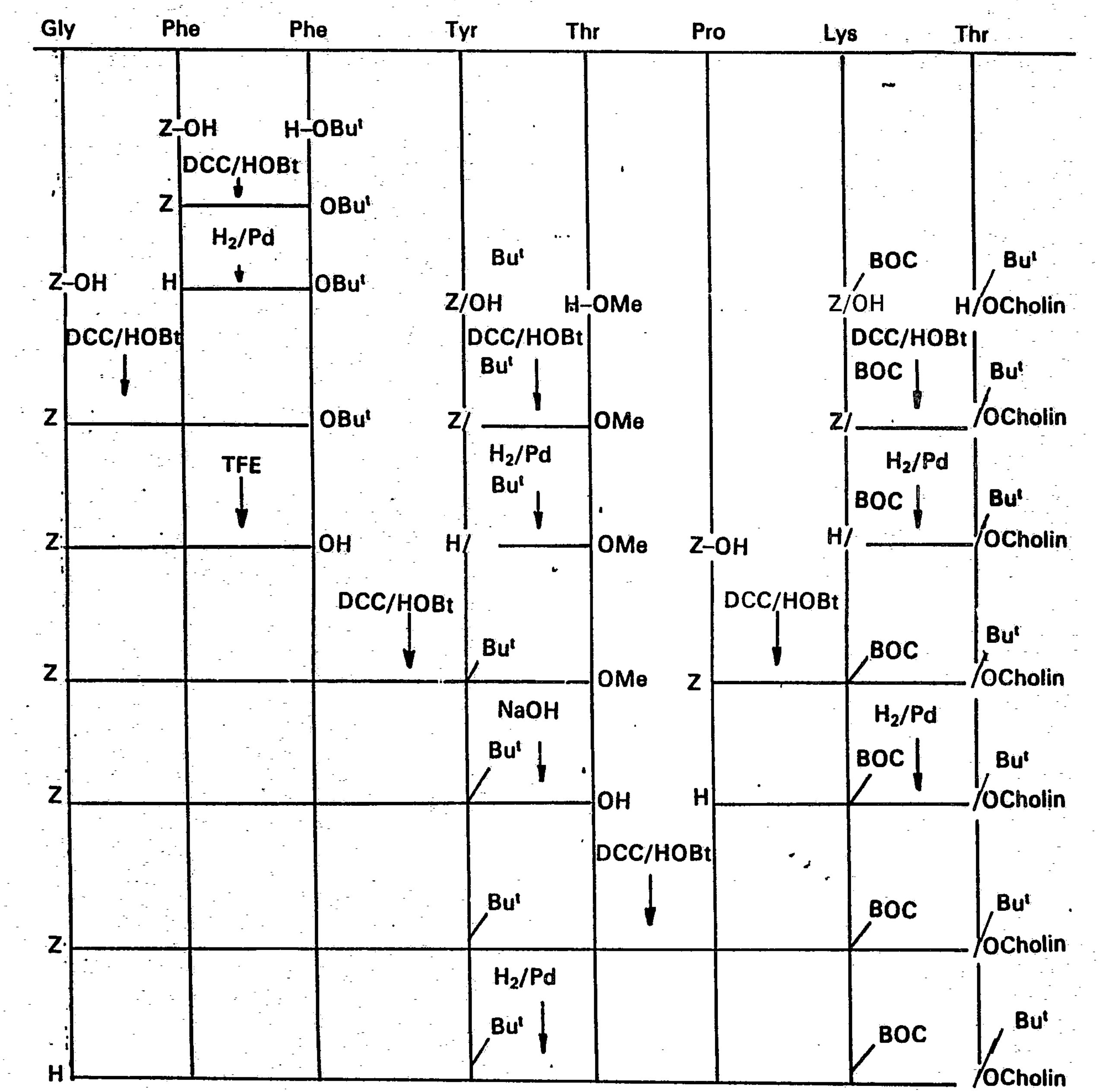


Aminosäure- und Elementaranalyse entsprechen der Theorie

Herstellungsbeispiel 2
Schweineinsulin-Arg$^{B31}$-Arg$^{B32}$–OH durch tryptische Verdauung aus Schweineproinsulin

350 mg Schweineproinsulin werden in 25 ml 0,1 M Tris-HCl-Puffer, pH=7,5 gelöst. Zu dieser Lösung werden bei Raumtemperatur 500 µg Trypsin zugesetzt, wobei innerhalb weniger Stunden eine Trübung auftritt. Nach vollendeter Reaktion wird abzentrifugiert, der Niederschlag sauer gelöst und mit Hilfe von Acetatfolienelektrophorese oder HPLC analysiert. Nach erneuter Ausfällung und Waschen des Niederschlages wird dieser in an sich bekannter Weise aufgearbeitet oder

er wird an einem Kationenaustauscher mit 0,05 M Acetat, pH=4,0, mit einem Kochsalzgradienten bis 0,5 M gereinigt. Die entsprechenden Fraktionen werden vereinigt und ausgefällt. Nach Waschen und Umkristallisation werden 104 mg nach Aminosäureanalyse identifiziertes und nach HPLC und isoelektrischer Fokussierung als einheitlich charakterisiertes Schweineinsulin-$Arg^{B31}$-$Arg^{B32}$–OH isoliert.

In analoger Weise wird aus dem entsprechenden, in Position B31 modifizierten Schweineproinsulin das Insulin-Derivat Schweineinsulin-$Lys^{B31}$-$Arg^{B32}$–OH erhalten.

Arzneimittel

Beispiel 1

Insulin-$Arg^{B31}$-$Arg^{B32}$–OH vom Schwein (hergestellt durch tryptische Verdauung aus Proinsulin vom Schwein) in schwach saurer, gelöster Formulierung mit 40 I.E. pro ml und deren Depot-Wirksamkeit:

Man löst in einem Gesamtvolumen von 10 ml Wasser

| | |
|---|---|
| Insulin-$Arg^{B31}$-$Arg^{B32}$–OH vom Schwein (27 I.E./mg) | 14,8 mg |
| Traubenzucker (Monohydrat) krist. | 540,0 mg |
| p-Hydroxybenzoesäuremethylester | 10,0 mg |

Der pH-Wert wird durch Zugabe von 1N HCl bzw. 1 N NaOH auf pH=4,5 eingestellt.

Eine solche Lösung zeigt bei einer Dosierung von 0,4 I.E./kg am Kaninchen eine ausgeprägte Depot-Wirksamkeit. Die Fläche unter der Blutzuckerkurve ist gleich wie die eines Standardpräparates mit 40 I.E./ml.

Beispiel 2

Humaninsulin-(B30)-Cholinester, hergestellt durch Semisythese aus Schweineinsulin, in neutraler Formulierung mit 40 I.E. pro ml und deren Depot-Wirksamkeit:

Man löst in einem Gesamtvolumen von 10 ml Wasser

| | |
|---|---|
| Humaninsulin-(B30)-Cholinester (28 I.E./mg) | 14,3 mg |
| Natriumdihydrogenphosphat-dihydrat | 21,0 mg |
| m-Kresol | 27,0 mg |
| Glycerin | 160,0 mg |

Der pH-Wert wird durch Zugabe von 1N HCl bzw. 1 N NaOH auf pH=7,3 eingestellt.

Eine solche Suspension zeigt bei einer Dosierung von 0,4 I.E./kg am Kaninchen eine ausgeprägte Depot-Wirksamkeit.

Beispiel 3

Humaninsulin-$Arg^{B31}$–OH hergestellt aus Schweineinsulin durch Semisynthese, in Form einer kristallinen NPH-Zubereitung mit 40 I.E./ml und deren stark verzögerte Wirkung:

Man löst in einem Gesamt-Volumen von 10 ml mit Wasser:

| | |
|---|---|
| Humaninsulin-$Arg^{B31}$–OH (27,5 I.E./mg) | 14,5 mg |
| Protaminsulfat | 1,3 mg |
| Natriumdihydrogenphosphat-dihydrat | 21,0 mg |
| m-Kresol | 15,0 mg |
| Phenol | 6,0 mg |
| Glycerin | 160,0 mg |

Der pH-Wert wird durch Zugabe von 1 N HCl bzw. 1 N NaOH auf pH=7,3 eingestellt.

Eine solche Kristallsuspension zeigt bei einer Dosierung von 0,4 I.E./kg am Kaninchen eine stark verzögerte Wirkung.

Beispiel 4

Mischung von Humaninsulin-$Arg^{B31}$–OH und Humaninsulin-$Arg^{B31}$-$Arg^{B32}$–OH, beide hergestellt durch Semisynthese aus Schweineinsulin, in Form einer zinkhaltigen Suspension mit 40 I.E./ml und deren stark verzögerte Wirkung:

Man löst in einem Gesamt-Volumen von 10 ml mit Wasser:

| | |
|---|---|
| Humaninsulin-$Arg^{B31}$–OH (27,5 I.E./mg) | 7,3 mg |
| Humaninsulin-$Arg^{B31}$-$Arg^{B32}$–OH 27,0 I.E./mg) | 7,4 mg |
| Zinkchlorid, wasserfrei | 0,46 mg |
| Natriumacetat | 14,0 mg |
| p-Hydroxybenzoesäuremethylester | 10,0 mg |
| Kochsalz | 80,0 mg |

Der pH-Wert wird durch Zugabe von 1 N HCl bzw. 1 N NaOH auf pH=7,0 eingestellt.

Eine solche Suspension zeigt bei einer Dosierung von 0,4 I.E./kg am Kaninchen eine stark verzögerte Wirkung.

Beispiel 5

Humaninsulin-$Arg^{B31}$-$Lys^{B32}$–$OCH_3$ hergestellt durch Semisynthese aus Schweineinsulin, in Form einer schwach sauren, gelösten Zubereitung mit 100 I.E./ml und deren verzögerte Wirkung:

Man löst in einem Gesamt-Volumen von 10 ml mit Wasser:

| | |
|---|---|
| Humaninsulin-$Arg^{B31}$-$Lys^{B32}$–$OCH_3$ (27,0 I.E./mg) | 37,0 mg |
| Natriumacetat | 14,0 mg |
| p-Hydroxybenzoesäuremethylester | 10,0 mg |
| Kochsalz | 80,0 mg |

Durch Zugabe von 1 N HCl bzw. 1 N NaOH wird pH=6,0 eingestellt.

Eine solche Lösung zeigt am Kaninchen eine verzögerte Wirkung.

Beispiel 6

Humaninsulin-Arg-$^{B31}$–OH, hergestellt durch tryptische Spaltung aus Primaten-Präproinsulin

bakteriellen Ursprungs, in Form von NPH-Kristallen, in Mischung mit Humaninsulin-Arg-$^{B31}$-Arg$^{B32}$–OH, hergestellt durch tryptische Spaltung aus Primaten-Präproinsulin bakteriellen Ursprungs:

Man löst in einem Gesamt-Volumen von 10 ml mit Wasser:

| | |
|---|---|
| Humaninsulin-Arg$^{B31}$–OH (27,5 I.E./mg) | 11,1 mg |
| Humaninsulin-Arg-$^{B31}$-Arg$^{B32}$–OH (27,0 I.E./mg) | 3,7 mg |
| Protaminsulfat | 1,0 mg |
| Natriumdihydrogenphosphat-2-Hydrat | 21,0 mg |
| m-Cresol | 15,0 mg |
| Phenol | 6,0 mg |
| Glycerin | 160,0 mg |

Der pH wird durch Zugabe von 1 N NaOH bzw. 1 N HCl auf pH=7,2 eingestellt.

Diese Suspension zeigt am Kaninchen (0,4 I.E./kg) eine stark verzögerte Wirkung.

**Patentansprüche für die Vertragsstaaten: BE, CH, DE, FR, GB, IT, LI, LU, NL, SE**

1. Insulin-Derivat der Formel I,

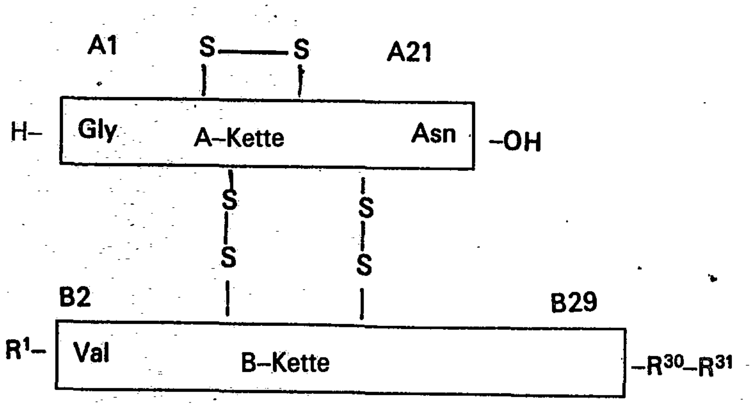

(I)

in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, wobei, falls $R^1$ für H–Phe steht, der C-Terminus-$R^{30}$-$R^{31}$ nicht -Thr-$(Arg)_m$–OH, -Ala-$(Arg)_m$–OH oder -Ser-$(Arg)_m$–OH mit m=1 oder 2 bedeuten kann, mit der Massgabe, dass auch Verbindungen ausgenommen sind, in denen zugleich $R^1$ H–Phe, $R^{30}$ Thr und $R^{31}$ a) eine Aminosäure, b) ein Peptid oder c) ein Amid oder Ester von a) oder b) ist, gekennzeichnet durch einen isoelektrischen Punkt zwischen 5,8 und 8,5, oder dessen physiologisch verträglichen Salze.

2. Insulin-Derivat der Formel I gemäss Anspruch 1, dadurch gekennzeichnet, dass

$R^{31}$ für einen Rest der Formel $-X_nS$ steht, in welchem

n = 0, 1, 2 oder 3 ist,

X für gleiche oder verschiedene Reste natürlich vorkommender neutraler oder basischer L-Aminosäuren und/oder der diesen entsprechenden D-Aminosäuren steht und

b) $R^1$ H–Phe bedeutet und $R^{30}$ Thr ist,

$R^{31}$ b 1) wie unter a 1) definiert ist,

b 2) wie unter a 2.3) definiert ist,

b 3) wie unter a 3.2) oder 3.3) definiert ist, oder

c) $R^1$ H–Phe bedeutet und $R^{30}$ der Rest einer anderen neutralen, genetisch kodierbaren L-Aminosäure als Thr ist,

$R^{31}$ c 1) wie unter a 1) definiert ist,

c 2.1) wie unter a 2.1) definiert ist,

c 2.2) Lys–OH, D-Lys–OH, D-Arg–OH, Hyl–OH, D-Hyl–OH, Orn–OH, D-Orn–OH, Cit–OH, D-Cit–OH, His–OH oder D-His–OH bedeutet,

c 2.3) für $X^B$-S' steht, worin S' die Bedeutung von S mit Ausnahme der von OH hat,

c 2.4) wie unter a 2.3) definiert ist,

c 3.1) für –X–X'–OH oder –X'–X–OH steht, worin X' wie unter c 2.2) definiert ist,

c 3.2) für $X_2$–S' steht,

c 3.3) wie unter a 3.1) definiert ist, wobei n=3 ist,

c 3.4) wie unter a 3.2) oder a 3.3) definiert ist, oder dessen physiologisch verträglichen Salze.

S OH oder eine physiologisch verträgliche, die Carboxygruppe blockierende Gruppe bedeutet, die falls n=0 ist, einen positiv geladenen oder protonierbaren basischen Rest trägt oder, falls n>0 ist, einen solchen Rest tragen kann und worin der C-Terminus

–X–S auch für den Rest einer zum entsprechenden Alkohol reduzierten Aminosäure oder, im Falle n=2 oder 3, für den Homoserinlacton-Rest stehen kann.

3. Insulin-Derivat der Formel I, in welcher

$R^{30}$ für den Rest einer neutralen, genetisch kodierbaren L-Aminosäure steht,

a) $R^1$ H bedeutet und

$R^{31}$ a 1) eine physiologisch verträgliche, die Carboxygruppe blockierende Gruppe $S^B$, die einen positiv geladenen oder protonierbaren basischen Rest trägt, bedeutet,

a 2.1) für $X^N$–$S^B$ steht, worin $X^N$ den Rest einer natürlich vorkommenden neutralen L-Aminosäure oder deren D-Form bedeutet,

a 2.2) für $X^B$–S steht, worin $X^B$ den Rest einer natürlich vorkommenden basischen L-Aminosäure oder deren D-Form und S OH oder eine die Carboxygruppe blockierende, gegebenenfalls einen positiv geladenen oder protonierbaren basischen Rest tragende Gruppe bedeuten,

a 2.3) für den Rest Y einer zum entsprechenden Alkohol reduzierten basischen Aminosäure $X^B$ steht,

a 3.1) für $-X_n$–S steht, worin N=2 oder 3 ist, X die Reste $X^N$ und/oder $X^B$ bedeutet und S, falls alle Reste $X=X^N$ sind, nur $S^B$ bedeuten kann,

a 3.2) für $-X_n$–Y steht, worin n=1 oder 2 ist,

a 3.3) für $-X^B$–Z, $-X^B$–$X^N$–Z, $-X^N$–$X^B$–Z oder $-X^B$–$X^B$–Z steht, worin Z=Y ist oder den Homoserinlacton-Rest bedeutet.

4. Insulin-Derivat nach einem der Ansprüche 1 bis 3, dadurch gekennzeichnet, dass $R^1$ für H–Phe steht.

5. Insulin-Derivat nach einem der Ansprüche 1 bis 4, dadurch gekennzeichnet, dass $R^{30}$ für Ala, Thr oder Ser steht.

6. Insulin-Derivat nach einem der Ansprüche 1 bis 5, dadurch gekennzeichnet, dass die A-Kette und die Kette (B2–29) die Sequenz des Humaninsulins aufweisen.

7. Insulin-Derivat nach einem der Ansprüche 1 bis 6, dadurch gekennzeichnet, dass die Aminosäurereste X, $X^N$ und $X^B$ sowie die Reste Y und Z in der L-Konfiguration vorliegen.

8. Insulin-Derivat nach einem der Ansprüche 2 bis 7, dadurch gekennzeichnet, dass S bzw. S' für ($C_1$ bis $C_6$)-Alkoxy, ($C_3$ bis $C_6$)-Cycloalkyloxy, $NH_2$, ($C_1$ bis $C_6$)-Alkylamino, Di-($C_1$ bis $C_6$)-alkylamino, Amino-($C_2$ bis $C_6$)-alkoxy, ($C_1$ bis $C_4$)-Alkylamino-($C_2$ bis $C_6$)-alkoxy, Di-($C_1$ bis $C_4$)-alkylamino-($C_2$ bis $C_6$)-alkoxy, Tri-($C_1$ bis $C_4$)-ammonio-($C_2$ bis $C_6$)-alkoxy, Amino-($C_2$ bis $C_6$)-alkylamino, [($C_1$ bis $C_4$)-Alkylamino]-($C_2$ bis $C_6$)-alkylamino, [Di-($C_1$ bis $C_4$)-alkylamino]-($C_2$ bis $C_6$)-alkylamino oder [Tri-($C_1$ bis $C_4$)alkylammonio]-($C_2$ bis $C_6$)-alkylamino steht und $S^B$ eine der acht letztgenannten Bedeutungen hat.

9. Verfahren zur Herstellung eines Insulin-Derivats der Formel I gemäss einem der Ansprüche 1 bis 8, dadurch gekennzeichnet, dass man

a) ein Des-Octapeptid (B23–30)-Insulin der Formel II,

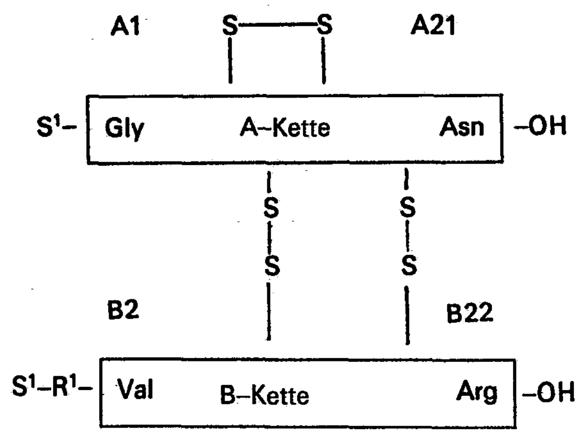


(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder den Methylsulfonylethyloxycarbonyl-(Msc)-Rest bedeuten, kondensiert mit einem Peptid der Formel III

H-Gly-Phe-Phe-Tyr($S^2$)-Thr($S^2$)-Pro-Lys($S^3$)-$R^{30}$-$R^{31}$ (III)

in welcher $R^{30}$ und $R^{31}$ die in den Ansprüchen 1 und 2 definierten Bedeutungen haben, $S^2$ für Wasserstoff, Bzl oder $Bu^t$ und $S^3$ für eine Urethanschutzgruppe, wie Boc, Moc, Fmoc oder Z stehen, wobei in den Resten $R^{30}$ und $R^{31}$ vorhandene freie COOH–, OH–, SH–, $NH_2$–, Guanidino- und/oder Imidazol-Gruppen, falls erforderlich, in an sich bekannter Weise geschützt vorliegen und gegebenenfalls vorhandene Schutzgruppen in an sich bekannter Weise abspaltet,

b) ein Des-B30-Insulin der Formel I, in welcher $R^1$ für H oder H–Phe und der C-Terminus $R^{30}$–$R^{31}$ zusammen für OH stehen, in Gegenwart von Trypsin oder einer trypsinähnlichen Endopeptidase umsetzt mit einer Verbindung der Formel IV

H–$R^{30}$–$R^{31}$ (IV)

in der $R^{30}$ und $R^{31}$ die in den Ansprüchen 1 bis 3 definierten Bedeutungen haben und worin vorhandene freie COOH–, OH–, SH–, ω–$NH_2$–, Guanidino- und/oder Imidazol-Funktionen, falls erforderlich, in an sich bekannter Weise geschützt vorliegen und anschliessend gegebenenfalls vorhandene Schutzgruppen in an sich bekannter Weise abspaltet, oder

c) zur Herstellung eines Insulin-Derivates gemäss Anspruch 7 ein Proinsulin, Proinsulinderivat, Präproinsulinderivat oder ein Intermediat dieser Verbindungen chemisch und/oder enzymatisch spaltet, und die nach a)–c) erhaltenen

Verbindungen gegebenenfalls in ihre physiologisch verträglichen Salze überführt.

10. Insulin-Derivat der Formel

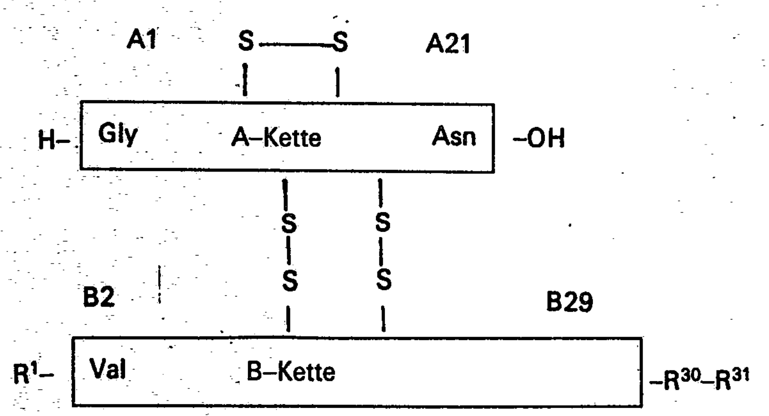

(I)

in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, wobei, falls $R^1$ für H–Phe steht, der C-Terminus $-R^{30}-R^{31}$ nicht -Thr-$(Arg)_m$–OH, -Ala-$(Arg)_m$–OH oder -Ser-$(Arg)_m$–OH mit m=1 oder 2 bedeuten kann, gekennzeichnet durch einen isoelektrischen Punkt zwischen 5,8 und 8,5, oder dessen physiologisch verträglichen Salze, bzw. ein solches gemäss einem oder mehreren der Ansprüche 1 bis 8 zur Verwendung als Heilmittel, insbesondere zur Behandlung des Diabetes mellitus.

11. Arzneimittel enthaltend ein Insulinderivat gemäss Anspruch 10.

12. Arzneimittel aus einem pharmazeutisch unbedenklichen Träger und einem Wirkstoff, dadurch gekennzeichnet, dass es als Wirkstoff ein Insulin-Derivat mit einem isoelektrischen Punkt zwischen 5,8 und 8,5 und der Formel I, in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, oder eines seiner physiologisch verträglichen Salze enthält.

13. Mittel gemäss Anspruch 12, dadurch gekennzeichnet, dass es als Wirkstoff Insulin-B31-Arg–OH oder Insulin-B31-Arg-Arg–OH, Ester oder Amide oder die physiologisch verträglichen Salze davon enthält

14. Mittel gemäss Anspruch 12 oder 13, dadurch gekennzeichnet, dass dieses einen pH-Wert zwischen 2,5 und 8,5 aufweist, ein geeignetes Isotonie-Mittel und ein geeignetes Konservierungsmittel enthält und dass das Insulin-Derivat der Formel I gelöst und/oder in Suspension vorliegt.

15. Mittel gemäss einem oder mehreren der Ansprüche 12 bis 14, dadurch gekennzeichnet, dass dieses einen geeigneten Puffer enthält und der pH-Wert zwischen 5,0 und 8,5 liegt.

16. Mittel gemäss einem oder mehreren der Ansprüche 12 bis 15, dadurch gekennzeichnet, dass dieses zwischen 0 und 100 µg Zink/100 I.U. enthält.

17. Mittel gemäss einem oder mehreren der Ansprüche 12 bis 16, dadurch gekennzeichnet, dass das Peptid der Formel I in Form eines Alkalisalzes oder des Ammoniumsalzes vorliegt.

18. Mittel gemäss einem oder mehreren der Ansprüche 12 bis 17, dadurch gekennzeichnet, dass ein beliebiger Anteil eines oder mehrerer Insulin-Derivate der Formel I oder ein Insulin-Derivat der Formel I in einer Mischung weiterer dieser Insulin-Derivate unabhängig voneinander jeweils in gelöster, amorpher und/oder kristalliner Form vorliegen.

19. Mittel gemäss einem oder mehreren der Ansprüche 12 bis 18, dadurch gekennzeichnet, dass dieses eine geeignete Menge eines Hilfsmittels mit verzögernder Wirkung enthält.

20. Mittel gemäss Anspruch 19, dadurch gekennzeichnet, dass dieses Verzögerungsprinzip in Kombination mit dem gesamten Wirkstoffanteil oder mit Teilen davon oder einem oder mehreren Insulin-Derivaten der Formel I in einer Mischung angewandt wird.

21. Mittel gemäss einem oder mehreren der Ansprüche 12 bis 20, dadurch gekennzeichnet, dass es verschiedene Insulin-Derivate der Formel I in Kombination mit mehreren verschiedenen verzögernd wirkenden Hilfsstoffen enthält.

22. Verfahren zur Herstellung eines Mittels gemäss Anspruch 11 oder 12, dadurch gekennzeichnet, dass man ein Insulin-Derivat der Formel I in eine geeignete Darreichungsform bringt.

**Patentansprüche für den Vertragsstaat AT**

1. Verfahren zur Herstellung eines Insulin-Derivats der Formel I

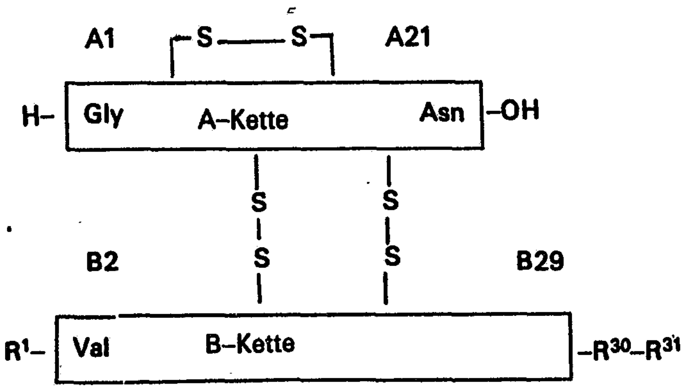

(I)

in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, wobei, falls $R^1$ für H–Phe steht, der C-Terminus-$R^{30}$–$R^{31}$ nicht -Thr(Arg)$_m$–OH, -Ala-(Arg)$_m$–OH oder -Ser-(Arg)$_m$–OH mit m=1 oder 2 bedeuten kann mit der Massgabe, dass auch Verbindungen ausgenommen sind, in denen zugleich $R^1$H–Phe, $R^{30}$ Thr und $R^{31}$ a) eine Aminosäure, b) ein Peptid oder c) ein Amid oder Ester von a) oder b) ist, mit einem isoelektrischen Punkt zwischen 5,8 und 8,5, oder dessen physiologisch verträglichen Salze, dadurch gekennzeichnet, dass man

a) ein Des-Octapeptid (B23–30)-Insulin der Formel II,

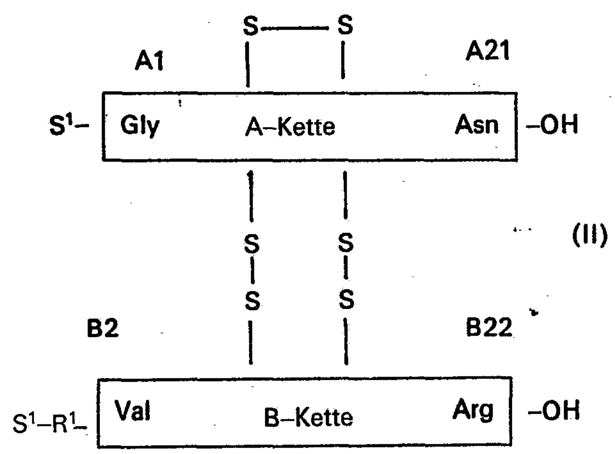

(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder den Methylsulfonylethyloxycarbonyl-(Msc)-Rest bedeuten, kondensiert mit einem Peptid der Formel III

H-Gly-Phe-Phe-Tyr($S^2$)-Thr($S^2$)-Pro-Lys($S^3$)-$R^{30}$-$R^{31}$ (III)

in welcher $R^{30}$ oder $R^{31}$ die obigen Bedeutungen haben, $S^2$ für Wasserstoff, Bzl oder $Bu^t$ und $S^3$ für eine Urethanschutzgruppe, wie Boc, Moc, Fmoc oder Z stehen, wobei in den Resten $R^{30}$ und $R^{31}$ vorhandene freie COOH–, OH–, SH–, $NH_2$–, Guanidino und/oder Imidazol-Gruppen, falls erforderlich, in an sich bekannter Weise geschützt vorliegen und gegebenenfalls vorhandene Schutzgruppen in an sich bekannter Weise abspaltet, oder

b) ein Des-B30-Insulin der Formel I, in welcher $R^1$ für H oder H-Phe und der C-Terminus $R^{30}$-$R^{31}$ zusammen für OH stehen, in Gegenwart von Trypsin oder einer trypsinähnlichen Endopeptidase umsetzt mit einer Verbindung der Formel IV

H–$R^{30}$–$R^{31}$ (IV)

in der $R^{30}$ und $R^{31}$ die in den Ansprüchen 1 bis 3 definierten Bedeutungen haben und worin vorhandene freie COOH–, OH–, SH–, ω–$NH_2$–, Guanidino- und/oder Imidazol-Funktionen, falls erforderlich, in an sich bekannter Weise geschützt vorliegen und anschliessend gegebenenfalls vorhandene Schutzgruppen in an sich bekannter Weise abspaltet, und die nach a) oder b) erhaltenen Verbindungen gegebenenfalls in ihre physiologisch verträglichen Salze überführt.

2. Verfahren gemäss Anspruch 1, dadurch gekennzeichnet, dass man ein Insulin-Derivat der Formel I herstellt, in welcher

$R^{31}$ für einen Rest der Formel –$X_n$S steht, in welchem

n= 0, 1, 2 oder 3 ist,

X für gleiche oder verschiedene Reste natürlich vorkommender neutraler oder basischer L-Aminosäuren und/oder der diesen entsprechenden D-Aminosäuren steht und

S OH oder eine physiologisch verträgliche, die Carboxygruppe blockierende Gruppe bedeutet,

die falls n=0 ist, einen positiv geladenen oder protonierbaren basischen Rest trägt oder, falls n>0 ist, einen solchen Rest tragen kann und worin der C-Terminus

–X–S auch für den Rest einer zum entsprechenden Alkohol reduzierten Aminosäure oder, im Falle n=2 oder 3, für den Homoserinlacton-Rest stehen kann.

3. Verfahren zur Herstellung eines Insulin-Derivats der Formel I, in welcher

$R^{30}$ für den Rest einer neutralen, genetisch kodierbaren L-Aminosäure steht,

a) $R^1$ H bedeutet und

$R^{31}$ a 1) eine physiologisch verträgliche, die Carboxygruppe blockierende Gruppe $S^B$, die einen positiv geladenen oder protonierbaren basischen Rest trägt, bedeutet,

a 2.1) für $X^N–S^B$, steht, worin $X^N$ den Rest einer natürlich vorkommenden neutralen L-Aminosäure oder deren D-Form bedeutet,

a 2.2) für $X^B–S$ steht, worin $X^B$ den Rest einer natürlich vorkommenden basischen L-Aminosäure oder deren D-Form und S OH oder eine die Carboxygruppe blockierende, gegebenenfalls einen positiv geladenen oder protonierbaren basischen Rest tragende Gruppe bedeuten,

a 2.3) für den Rest Y einer entsprechenden Alkohol reduzierten basischen Aminosäure $X^B$ steht,

a 3.1) für $–X_n–S$ steht, worin n=2 oder 3 ist, X die Reste $X^N$ und/oder $X^B$ bedeutet und S, falls alle Reste $X=N^N$ sind, nur $S^B$ bedeuten kann,

a 3.2) für $–X_n–Y$ steht, worin n=1 oder 2 ist,

a 3.3) für $–X^B–Z$, $–X^B–X^N–Z$, $–S^N–S^B–Z$ oder $–X^B–X^B–Z$ steht, worin Z=Y ist oder den Homoserinlacton-Rest bedeutet oder

b) $R^1$ H–Phe bedeutet und $R^{30}$ Thr ist,

$R^{31}$ b 1) wie unter a 1) definiert ist,

b 2) wie unter a 2.3) definiert ist,

b 3) wieunter a 3.2) oder 3.3) definiert ist, oder

c) $R^1$ H-Phe bedeutet und $R^{30}$ der Rest einer anderen neutralen, genetisch kodierbaren L-Aminosäure als Thr ist,

$R^{31}$ c 1) wie unter a 1) definiert ist,

c 2.1) wie unter a 2.1) definiert ist,

c 2.2) Lys–OH, D-Lys–OH, D-Arg–OH, Hyl–OH, D-Hyl–OH, Orn–OH, D-Orn–OH, Cit–OH, D-Cit–OH, His–OH oder D-His–OH bedeutet,

c 2.3) für $X^B–S'$ steht, worin S' die Bedeutung von S mit Ausnahme der von OH hat,

c 2.4) wie unter a 2.3) definiert ist,

c 3.1) für –X–X'–OH oder –X'–X–OH steht, worin X' wie unter c 2.2) definiert ist,

c 3.2) für $X_2–S'$ steht,

c 3.3) wie unter a 3.1) definiert ist, wobei n=3 ist,

c 3.4) wie unter a 3.2) oder a 3.3) definiert ist, oder dessen physiologisch verträglichen Salze, dadurch gekennzeichnet, dass man

a) ein Des-Octapeptid (B23–30)-Insulin der Formel II, Insulin-Derivat der Formel I,

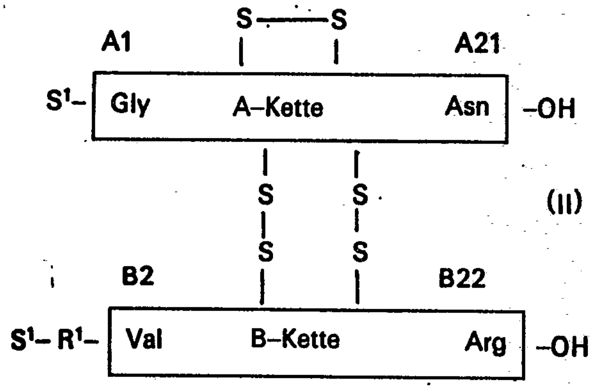

(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder denMethylsulfonylethyloxycarbonyl-(Msc)-Rest bedeuten, kondensiert mit einem Peptid der Formel III, in welcher $R^{30}$ oder $R^{31}$ die obigen Bedeutungen haben, $S^2$ für Wasserstoff, Bzl oder $Bu^t$ und $S^3$ für eine Urethanschutzgruppe, wie Boc, Moc, Fmoc oder Z stehen, wobei in den Resten $R^{30}$ und $R^{31}$ vorhandene freie COOH–, OH–, SH–, $NH_2$–, Guanidino und/oder Imidazol-Gruppen, falls erforderlich, in an sich bekannter Weise geschützt vorliegen und gegebenenfalls vorhandene Schutzgruppen in an sich bekannter Weise abspaltet, oder

b) ein Des-B30-Insulin der Formel I, in welcher $R^1$ für H oder H-Phe und der C-Terminus $R^{30}–R^{31}$ zusammen für OH stehen, in Gegenwart von Trypsin oder einer trypsinähnlichen Endopeptidase umsetzt mit einer Verbindung der Formel IV, in der $R^{30}$ und $R^{31}$ die in den Ansprüchen 1 bis 3 definierten Bedeutungen haben und worin vorhandene freie COOH–, OH–, SH–, $ω–NH_2$–, Guanidino- und/oder Imidazol-Funktionen, falls erforderlich, in an sich bekannter Weise geschützt vorliegen und anschliessend gegebenenfalls vorhandene Schutzgruppen in an sich bekannter Weise abspaltet, und die nach a) oder b) erhaltenen Verbindungen gegebenenfalls in ihre physiologisch verträglichen Salze überführt.

4. Verfahren nach einem oder mehreren der Ansprüche 1–3, dadurch gekennzeichnet, dass man ein Insulin-Derivat der Formel I herstellt, in der $R^1$ für H–Phe steht.

5. Verfahren nach einem oder mehreren der Ansprüche 1 bis 4, dadurch gekennzeichnet, dass man ein Insulin-Derivat der Formel I herstellt, in der $R^{30}$ für Ala, Thr oder Ser steht.

6. Verfahren nach einem oder mehreren der Ansprüche 1 bis 5, dadurch gekennzeichnet, dass man ein Insulin-Derivat der Formel I herstellt, in der die A-Kette und die Kette (B2–29) die Sequenz des Humaninsulins aufweisen.

7. Verfahren nach einem oder mehreren der Ansprüche 1 bis 6, dadurch gekennzeichnet, dass man ein Insulin-Derivat der Formel I herstellt, in der die Aminosäurereste, X, $X^N$ und $X^B$ sowie die Reste Y und Z in der L-Konfiguration vorliegen.

8. Verfahren zur Herstellung eines Insulin-Derivats der Formel I, in der die Aminosäurereste, X,

$X^N$, $X^B$, Y und Z definiert sind wie in Anspruch 7, dadurch gekennzeichnet, dass man ein Proinsulin, Proinsulinderivat, Präproinsulinderivat oder ein Intermediat dieser Verbindungen chemisch und/oder enzymatisch spaltet und gegebenenfalls in physiologisch verträgliche Salze überführt.

9. Verfahren nach einem oder mehreren der Ansprüche 2–8, dadurch gekennzeichnet, dass man ein Insulin-Derivat der Formel I herstellt, in der S bzw. S' für ($C_1$ bis $C_6$)-Alkoxy, ($C_3$ bis $C_6$)-Cycloalkoxy, $NH_2$, ($C_1$ bis $C_6$)-Alkylamino,Di-($C_1$ bis $C_6$)-alkylamino, Amino-($C_2$ bis $C_6$)-alkoxy, ($C_1$ bis $C_4$)-Alkylamino-($C_2$ bis $C_6$)-alkoxy, Di-($C_1$ bis $C_4$)-alkylamino-($C_2$ bis $C_6$)-alkoxy, Tri-($C_1$ bis $C_4$)-ammonio-($C_2$ bis $C_6$)-alkoxy, Amino-($C_2$ bis $C_6$)-alkylamino, [($C_1$ bis $C_4$)-Alkylamino]-($C_2$ bis $C_6$)-alkylamino, [Di-($C_1$ bis $C_4$)-alkylamino]-($C_2$ bis $C_6$)-alkylamino oder [Tri-($C_1$ bis $C_4$)-alkylammonio]-($C_2$ bis $C_6$)-alkylamino steht und $S^B$ eine der acht letztgenannten Bedeutungen hat.

10. Verfahren nach einem oder mehreren der Ansprüche 1 bis 9, dadurch gekennzeichnet, dass man dem Insulinderivat noch ein geeignetes Isotoniemittel und ein geeignetes Konservierungsmittel zusetzt, wobei das Insulinderivat der Formel I gelöst und/oder in Suspension vorliegt und das Produkt einen pH-Wert zwischen 2,5 und 8,5 aufweist.

11. Verfahren nach Anspruch 10, dadurch gekennzeichnet, dass man noch einen geeigneten Puffer zusetzt dergestalt, dass schliesslich der pH-Wert zwischen 5,0 und 8,5 liegt.

12. Verfahren nach Anspruch 10 oder 11, dadurch gekennzeichnet, dass man noch eine zusätzliche Depotwirkung durch Zugabe von Zink in einer solchen Menge erzeugt, dass das Produkt bis 100 µm Zink/100 I.E. enthält.

13. Verfahren nach einem oder mehreren der Ansprüche 10 bis 12, dadurch gekennzeichnet, dass das Insulinderivat der Formel I in Form eines Alkalisalzes oder des Ammoniumsalzes vorliegt.

14. Verfahren nach einem oder mehreren der Ansprüche 10 bis 13, dadurch gekennzeichnet, dass ein beliebiger Anteil eines oder mehrerer Insulin-Derivate der Formel I oder ein Insulin-Derivat der Formel I in einer Mischung weiterer dieser Insulin-Derivate unabhängig voneinander jeweils in gelöster, amorpher und/oder kristalliner Form vorliegen.

15. Verfahren nach einem oder mehreren der Ansprüche 10 bis 14, dadurch gekennzeichnet, dass eine geeignete Menge eines Hilfsmittels mit verzögernder Wirkung eingearbeitet wird.

16. Verfahren nach Anspruch 15, dadurch gekennzeichnet, dass dieses Verzögerungsprinzip in Kombination mit dem gesamten Wirkstoffanteil oder mit Teilen davon oder einem oder mehreren Insulin-Derivaten der Formel I in einer Mischung angewandt wird.

17. Verfahren nach einem der Ansprüche 15 oder 16, dadurch gekennzeichnet, dass eine Kombination mehrerer verschiedener verzögernd wirkender Hilfsstoffe eingearbeitet wird.

18. Verfahren von Insulin-Derivaten der Formel I gemäss Anspruch 1, in welcher

$R^1$ H oder H–Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, wobei $R^1$ für H–Phe steht und der C-Terminus-$R^{30}$–$R^{31}$ α)-Thr(Arg)$_m$–OH, -Ala-(Arg)$_m$OH oder -Ser-(Arg)$_m$–OH mit m=1 oder 2 oder β) $R^{30}$ Thr und $R^{31}$ a) eine Aminosäure, b) ein Peptid oder c) ein Amid oder Ester von a) oder b) ist, vorzugsweise jedoch von Insulin-B31-Arg–OH oder Insulin-B31-Arg-Arg–OH, eines Esters oder Amids oder eines physiologisch verträglichen Salzes davon zur Herstellung von Arzneimitteln gegen Diabetes mellitus.

**Revendications pour les Etats contractants BE, CH, DE, FR, GB, IT, LI, LU, NL, SE**

1. Dérivé d'insuline de formule I

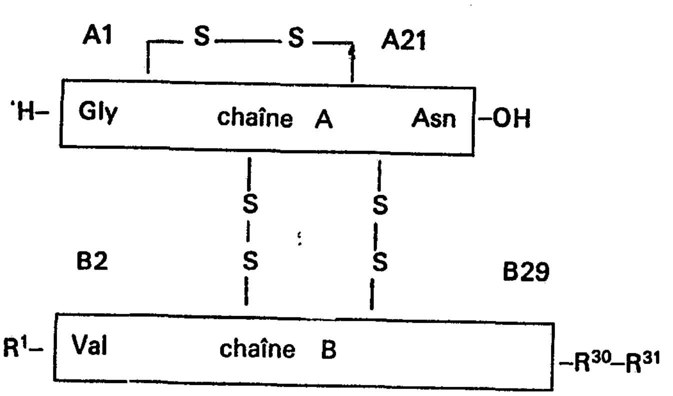

(I)

dans laquelle

$R^1$ signifie H ou H–Phe,

$R^{30}$ représente le reste d'un L-aminoacide neutre génétiquement codable et

$R^{31}$ représente un groupe organique physiologiquement inoffensif de caractère basique, ayant jusqu'à 50 atomes de C, et à la structure duquel participent 0 à 3α-aminoacide(s) et dont la fonction carboxy terminal éventuellement présente peut se trouver sous forme libre, sous forme de fonction ester, sous forme de fonction amine, sous forme de lactone ou réduite en $CH_2OH$, l'extrémité C-terminal $R^{30}$ ou $R^{31}$, au cas où $R^1$ représente H-Phe, ne pouvant signifier Thr$(Arg)_m$–OH, -Ala-$(Arg)_m$–OH, ou -Ser$(Arg)_m$–OH avec m=1 ou 2, à la condition que soient exclus aussi les composés dans lesquels à la fois $R^1$ est H-Phe, $R^{30}$ est Thr est $R^{31}$ et a) un aminoacide, b) un peptide ou c), un amide ou un ester de a) ou b), caractérisé par un point isoélectrique compris entre 5,8 et 8,5, ou ses sels physiologiquement acceptables.

2. Dérivé de l'insuline de formule I selon la revendication 1, caractérisé en ce que

$R^{31}$ est un radical de formule $-X_nS$ dans laquelle n=0, 1, 2 ou 3,

X représente des restes identiques ou différents de L-aminoacides neutres ou basiques naturels et/ou des D-aminoacides correspondant à ceux-ci et

S désigne OH ou un groupe physiologiquement acceptable bloquant le groupe carboxy, qui, au cas où n=0, porte un radical basique positivement chargé ou protonable, et, au cas où n est supérieur à 0, peut porter un tel reste, l'extrémité C-terminal –X–S pouvant représenter aussi le reste d'un aminoacide réduit en alcool correspondant ou bien, au cas où n=2 ou 3, le reste homoserinelactone.

3. Dérivé de l'insuline de formule I dans laquelle

$R^{30}$ représente le reste d'un L-aminoacide neutre génétiquement codable,

a) $R^1$ désigne H et

$R^{31}$ a 1) désigne un groupe $S^B$ physiologiquement acceptable bloquant le groupe carboxy, qui porte un reste basique positivement chargé ou protonable,

a 2.1) représente $X^N$–$S^B$, $X^N$ désignant le reste d'un L-aminoacide neutre naturel ou de sa forme D,

a 2.2) représente $X^B$–S, $X^B$ désignant le reste d'un L-aminoacide basique naturel ou de sa forme D et S désignant OH ou un groupe bloquant le groupe carboxy, portant éventuellement un reste basique positivement chargé ou protonable,

a 2.3) représente le reste Y d'un aminoacide basique,

$X^B$ réduit en alcool correspondant,

a 3.1) représente $-X_n$–S, n=2 ou 3, X désignant le radical $X^N$ et/ou $X^B$ et S, au cas où tous les radicaux $X=X^N$, ne pouvant signifier que $S^B$,

a 3.2) représente $-X_n$–Y, où n=1 ou 2,

a 3.3) représente $X^B$–Z, $-X^B$–$X^N$–Z, $-X^N$–$X^B$–Z ou $-X^B$–$X^B$–Z, Z étant égal Y ou désignant le reste homoserinelactone ou

b) $R^1$ désigne H-Phe et $R^{30}$ est Thr,

$R^{31}$ b 1) est défini comme en a 1),

b 2) est défini comme en 2.3),

b 3) est défini comme en a 3.2) ou 3.3), ou

c) $R^1$ est H–Phe et $R^{30}$ est le reste d'un L-aminoacide génétiquement codable neutre autre que Thr,

$R^{31}$ c 1) est défini comme en a 1),

c 2.1) est défini comme en a 2.1),

c 2.2) désigne Lys–OH, D-Lys–OH, D-Arg–OH, Hyl–OH, D-Hyl–OH, Orn–OH, D-Orn–OH, Cit–OH, D-Cit–OH, His–OH ou D-His–OH,

c 2.3) représente $X^B$–S', S' ayant la significatif de S à l'exception de OH,

c 2.4) est défini comme en a 2.3),

c 3.1) représente X–X'–OH ou X'–X–OH, X' étant défini comme c 2.2),

c 3.2) représente $X_2$–S',

c 3.3) est défini comme dans a 3.1), n=3,

c 3.4) est défini comme a 3.2) ou a 3.3), ou ses sels pharmacologiquement acceptables.

4. Dérivé de l'insuline suivant l'une des revendications 1 à 3, caractérisé en ce que $R^1$ représente H-Phe.

5. Dérivé de l'insuline suivant l'une des revendications 1 à 4, caractérisé en ce que $R^{30}$ est Ala, Thr ou Ser.

6. Dérivé de l'insuline suivant l'une des revendications 1 à 5, caractérisé en ce que la chaîne A et la chaîne (B2–29) présente la séquence de l'insuline humaine.

7. Dérivé de l'insuline selon l'une des revendications 1 à 6, caractérisé en ce que les restes d'aminoacide X, $X^N$ et $X^B$ ainsi que les restes Y et Z se trouvent sous la configuration L.

8. Dérivé de l'insuline selon l'une des revendications 2 à 7, caractérisé en ce que S ou S' représentent ($C_1$ à $C_6$)-alkoxy, ($C_3$ à $C_6$)-cycloalkoxy, $NH_2$, ($C_1$ à $C_6$)-alkylamino, di-($C_1$- à $C_6$)-alkylamino, amino-($C_2$ à $C_6$)-alkoxy, ($C_1$ à $C_4$)-alkylamino-($C_2$ à $C_6$)-alkoxy, di-($C_1$ à $C_4$)-alkylamino-($C_2$ à $C_6$)-alkoxy, tri-($C_1$ à $C_4$)-ammonio-($C_2$ à $C_6$)-alkoxy, amino-($C_2$ à $C_6$)-alkylamino, [($C_1$ à $C_4$)-alkylamino]-($C_2$ à $C_6$)-alkylamino, [di-($C_1$ à $C_4$)-alkylamino]-($C_2$ à $C_6$)alkylamino ou [Tri-($C_1$ à $C_4$)alkylamino]-($C_2$ à $C_6$)-alkylamino et $S^B$ a l'une des 8 dernières significations indiquées.

9. Procédé de préparation d'un dérivé de l'insuline de formule I selon l'une des revendications 1 à 8, caractérisé en ce que

a) on condense une Des-octapeptide (B23–30)-insuline de formule II,

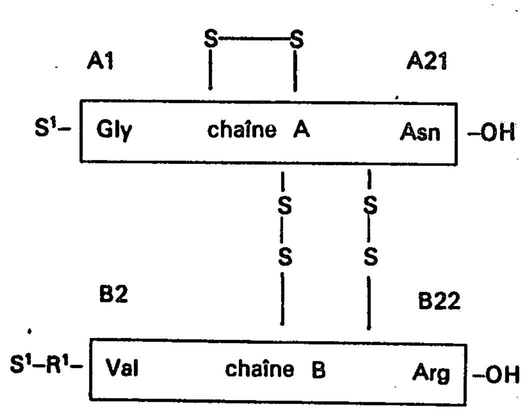

(II)

dans laquelle $R^1$ désigne Phe ou une liaison et $S^1$ désigne un groupe protecteur du groupe amino séparable par solvolyse protonique ou par élimination comme le radical tert-butyloxycarbonyle (Boc), tert-amyloxycarbonyle (Aoc) ou méthylsulfonyléthyloxycarbonyle (Msc) avec un peptide de formule III

H-Gly-Phe-Phe-Tyr($S^2$)-Thr($S^2$)-Pro-Lys($S^3$)-$R^{30}$-$R^{31}$ (III)

dans laquelle $R^{30}$ et $R^{31}$ ont les significations définies dans les revendications 1 et 2, $S^2$ représente l'hydrogène, Bzl ou $Bu^t$ et $S^3$ représentent un groupe protecteur du groupe uréthanne comme Boc, Moc, Fmoc ou Z, les groupes COOH–, OH–, SH–, $NH_2$–, guanidino et/ou imidazole libres présents dans les radicaux $R^{30}$ et $R^{31}$ étant, si c'est nécessaire, protégés de façon connue en soi et on sépare de façon connue en soi les groupes protecteurs éventuellement présents,

b) On fait réagir une Des-B30-insuline de formule I, dans laquelle $R^1$ représente H ou H–Phe et l'extrémité C-terminal $R^{30}$–$R^{31}$ représente OH, en présence de trypsine ou d'une endopeptidase analogue à la trypsine avec un composé de formule IV

H–$R^{30}$–$R^{31}$ (IV)

dans laquelle $R^{30}$ et $R^{31}$ ont les significations définies dans les revendications 1 à 3, les fonctions COOH–, OH–, SH–, ω–$NH_2$–, guanidino et/ou imidazol libre présente étant, si c'est nécessaire, protégées de façon connue en soi et ensuite on sépare les groupes protecteurs éventuellement présents de façon connue en soi, ou

c) pour préparer un dérivé de l'insuline selon la revendication 7, on coupe de façon chimique et/ou enzymatique une pro-insuline, un dérivé de pro-insuline, un dérivé de pré-pro-insuline ou un intermédiaire de ces composés et on transforme éventuellement les composés obtenus suivant a) à c) en leurs sels physiologiquement acceptables.

10. Dérivés de l'insuline de formule

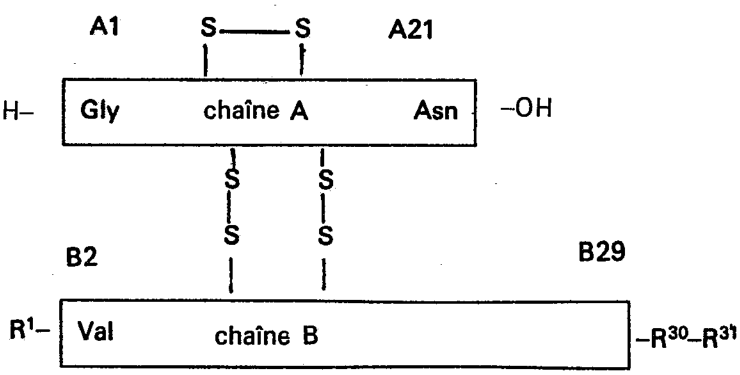

(I)

dans laquelle

$R^1$ désigne H ou H–Phe,

$R^{30}$ représente le radical d'un L-aminoacide neutre génétiquement codable et

$R^{31}$ représente un groupe organique physiologiquement inoffensif, de caractère basique, ayant jusqu'à 50 atomes de C, à la structure duquel participe 0 à 3 -aminoacide et dont la fonction carboxy terminale éventuellement présente peut être sous forme de fonction ester, de fonction amine, sous forme de lactone ou bien réduit en $CH_2OH$, l'extrémité C-terminal $R^{30}$–$R^{31}$, au cas où $R^1$ représente H–Phe, ne pouvant signifier -Thr-$(Arg)_m$–OH, -Ala-$(Arg)_m$–OH ou -Ser-$(Arg)_m$–OH avec m=1 ou 2, caractérisé par un point iso-électrique compris entre 5,8 et 8,5, ou ses sels pharmaceutiquement acceptables, ou un tel composé selon l'une ou plusieurs des revendications 1 à 8, destiné à l'emploi comme médicament, en particulier pour le traitement du diabète sucré.

11. Médicament contenant un dérivé de l'insuline selon la revendication 10.

12. Médicament constitué d'un véhicule pharmaceutiquement acceptable et d'une substance active, caractérisé en ce qu'il contient comme substance acetive un dérivé de l'insuline ayant un point iso-électrique compris entre 5,8 et 8,5 et de formule I, dans laquelle

$R^1$ désigne H ou H–Phe,

$R^{30}$ représente le radical d'un L-aminoacide neutre codable génétiquement et

$R^{31}$ représente un groupe organique physiologiquement inoffensif de caractère basique, ayant jusqu'à 50 atomes de C, à la structure duquel participent 0 à 3 α-aminoacide(s) et dont la fonction carboxylique terminale dans la fonction eventuellement presente se trouve sous forme libre, sous forme de fonction ester, de fonction amine, sous forme de lactone ou bien réduite en $CH_2OH$, ou un de ses sels physiologiquement acceptables.

13. Médicament selon la revendication 12, caractérisé en ce qu'il contient comme substance active insuline -B31-Arg–OH ou insuline -B31-Arg-Arg–OH, les esters ou amines ou les sels physiologiquement acceptables de ceux-ci.

14. Médicament selon la revendication 12 ou 13, caractérisé en ce qu'il présente un pH compris entre 2,5 et 8,5, un agent d'isotonicité approprié et un agent de conservation approprié et en ce que le dérivé de l'insuline de formule I est présent à l'état dissous et/ou en suspension.

15. Médicament suivant une ou plusieurs des revendications 12 à 14, caractérisé en ce qu'il

contient un tampon approprié et en ce que le pH est compris entre 5,0 et 8,5.

16. Médicament selon une ou plusieurs des revendications 12 à 15, caractérisé en ce qu'il contient entre 0 et 100 microgrammes de zinc par 100 UI.

17. Médicament selon une ou plusieurs des revendications 12 à 16, caractérisé en ce que le peptide de formule I est présent sous la forme d'un sel d'alcalin ou du sel d'ammonium.

18. Médicament selon une ou plusieurs des revendications 12 à 17, caractérisé en ce qu'une proportion quelconque d'un ou de plusieurs dérivés de l'insuline de formule I ou d'un dérivé de l'insuline de formule I se trouve en mélange avec d'autres de ces dérivés de l'insuline, chacun indépendamment l'un de l'autre, sous forme dissoute, amorphe et/ou cristalline.

19. Médicament selon une ou plusieurs des revendications 12 à 18, caractérisé en ce qu'il contient une quantité appropriée d'un adjuvant à effet retard.

20. Médicament selon la revendication 19, caractérisé en ce que ce principe retardant est employé en combinaison avec la totalité de la substance active ou avec des parties de celle-ci ou avec un ou plusieurs dérivés de l'insuline de formule I en mélange.

21. Médicament selon l'une ou plusieurs des revendications 12 à 20, caractérisé en ce qu'il contient différents dérivés de l'insuline de formule I en combinaison avec plusieurs adjuvants différents à effet retard.

22. Procédé de préparation d'un médicament suivant la revendication 11ou 12, caractérisé en ce que l'on met un dérivé de l'insuline de formule I sous une forme d'administration appropriée.

**Revendications pour l'Etat contractant AT**

1. Procédé de préparation d'un dérivé de l'insuline de formule I

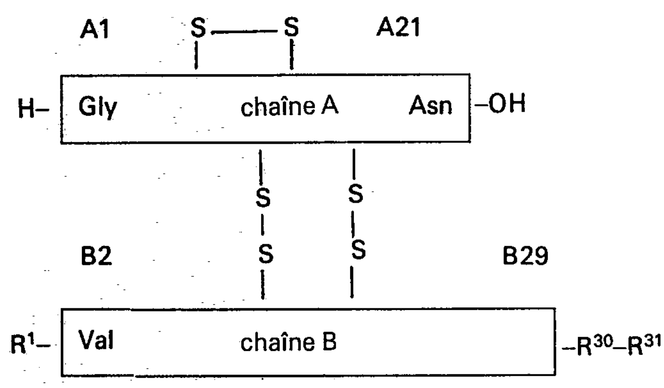

(I)

dans laquelle

$R^1$ signifie H ou H–Phe,

$R^{30}$ représente le reste d'un L-aminoacide neutre génétiquement codable et

$R^{31}$ représente un groupe organique physiologiquement inoffensif, de caractère basique, ayant jusqu'à 50 atomes de C, à la structure duquel participent 0 à 3 $\alpha$-aminoacide(s) et dont la fonction carboxy terminale éventuellement présente peut se trouver sous forme libre, sous forme de fonction ester, de fonction amine, sous forme de lactone ou bien réduite en $CH_2OH$–, l'extrémité C-terminal $R^{30}$–$R^{31}$, au cas où $R^1$ représente H-Phe, ne pouvant signifier Thr$(Arg)_m$–OH, -Ala$(Arg)_m$–OH ou -Ser$(Arg)_m$–OH avec m=1 ou 2, à condition que soient exclus aussi les composés dans lesquels à la fois $R^1$ est H–Phe, $R^{30}$ est Thr est $R^{31}$ et a) un aminoacide, b) un peptide ou c) un amide ou un ester de a) ou b), ayant un poids iso-électrique compris entre 5,8 et 8,5, ou ses sels physiologiquements acceptables, caractérisé en ce que

a) on condense un Des-octapeptide (B23–30)-insuline de formule II,

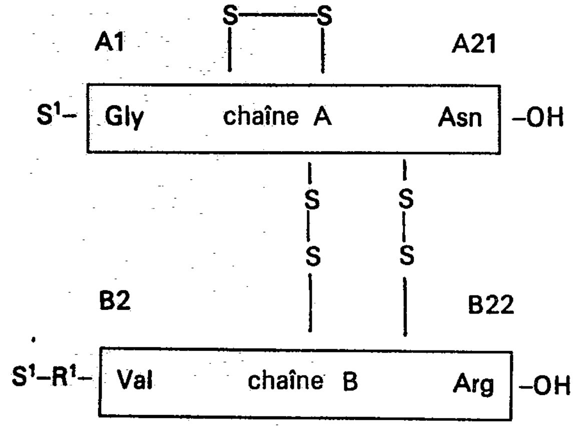

(II)

dans laquelle $R^1$ désigne Phe ou une liaison et S désigne un groupe protecteur du groupe amino qui peut être séparé par solvolyse protonique ou par – élimination comme le radical tert- butyloxycarbonyle (Boc), tert-amyloxycarbonyle (Aoc) ou méthylsulfonyléthylcarbonyle (Msc) avec un peptide de formule III

H-Gly-Phe-Phe-Tyr($S^2$)-Thr($S^2$)-Pro-Lys($S^3$)-$R^{30}$-$R^{31}$ (III)

dans laquelle $R^{30}$ et $R^{31}$ ont les significations ci-dessus, $S^2$ représente l'hydrogène, Bzl ou $Bu^t$ et $S^3$ représente un groupe protecteur du groupe uréthanne comme Boc, Moc, Fmoc ou Z, les groupes COOH–, OH–, SH–, $NH_2$, guanidino et/ou imidazole libres présents dans les radicaux $R^{30}$ et $R^{31}$ étant si nécessaire protégés de façon connue en soi, et on sépare de façon connue en soi les groupes protecteurs éventuellement présents, ou

b) on fait réagir un Des-B30-insuline de formule I, dans laquelle $R^1$ représente H ou H–Phe et l'extrémité C-terminal $R^{30}$–$R^{31}$ représente OH, en présence de trypsine ou d'une endopeptidase analogue à la trypsine, avec un composé de formule IV

H–$R^{30}$–$R^{31}$ (IV)

dans laquelle $R^{30}$ et $R^{31}$ ont les significations définies dans les revendications 1 à 3, les fonctions COOH–, OH–, SH–, ω–$NH_2$–, guanidino- et/ou imidazole libres présents étant, si c'est nécessaire, protégées de façon connue en soi et ensuite on sépare de façon connue en soi les groupes protecteurs éventuellement présents et on transforme éventuellement les composé obtenus selon a) ou b) en leurs physiologiquement acceptables.

2. Procédé selon la revendication 1, caractérisé en ce que l'on prépare un dérivé de l'insuline de formule I dans laquelle

$R^{31}$ représente un radical de formule $-X_nS$ dans laquelle

n=0, 1, 2 ou 3,

X représente des restes identiques ou différents de L-aminoacides neutres ou basiques naturels et/ou des D-aminoacides correspondant à ceux-ci et

S signifie OH ou un groupe physiologiquement acceptable bloquant le groupe carboxy qui, au cas où n=0, porte un reste basique positivement chargé ou protonable basique et, dans le cas où n est supérieur à 0, peut porter un tel reste, l'extrémité C-terminal –X–S– pouvant représenter aussi le reste d'un aminoacide réduit en alcool correspondant et, au cas où n=2 ou 3, le reste homoserinelactone.

3. Procédé de préparation d'un dérivé de l'insuline de formule I dans laquelle

$R^{30}$ représente le reste d'un L-aminoacide neutre génétiquement codable,

a) $R^1$ signifie H et

$R^{31}$ a 1) désigne un groupe $S^B$ physiologiquement acceptable bloquant le groupe carboxy, qui porte un reste positivement chargé ou protonable basique,

a 2.1) représente $X^N$–$S^B$, $X^N$ désignant le reste d'un L-aminoacide naturel neutre ou de sa forme D,

a 2.2) représente $X^B$–S, $X^B$ désignant le reste d'un L-aminoacide basique naturel ou de sa forme D et S désignant OH ou un groupe bloquant le groupe carboxy, portant éventuellement un reste positivement chargé ou protonable basique,

a 2.3) représente le reste Y d'un aminoacide basique $X^B$ réduit en l'alcool correspondant,

a 3.1) représente $-X_n$–S, n=2 ou 3, X désignant le radical $X^N$ et/ou $X^B$ et S, au cas où tous les radicaux $X=X^N$, ne pouvant signifier que $S^B$,

a 3.2) représente $-X_n$–Y, où n=1 ou 2,

a 3.3) représente $X^B$–Z, –$X^B$–$X^N$–Z, –$S^N$–$S^B$–Z, ou $X^B$–$X^B$–Z, Z étant égal à Y ou désignant le reste homoserinelactone ou

b) $R^1$ désigne H–Phe et $R^{30}$ est Thr,

$R^{31}$ b 1) est défini comme en a 1),

b 2) est défini comme en 2.3),

b 3) est défini comme en a 3.2) ou 3.3), ou

c) $R^1$ désigne H–Phe et $R^{30}$ est le reste d'un L-aminoacide neutre génétiquement codable autre que Thr,

$R^{31}$ c 1) est défini comme en a 1),

c 2.1) est défini comme en a 2.1),

c 2.2) désigne Lys–OH, D-Lys–OH, D-Arg–OH, Hyl–OH, D-Hyl–OH, Orn–OH, D-Orn–OH, Cit–OH, D-Cit–OH, His–OH ou D-His–OH,

c 2.3) représente $X^B$–S', S' ayant la significatif de S à l'exception de OH,

c 2.4) est défini comme en a 2.3),

c 3.1) représente X–X'–OH ou X'–X–OH, X' étant défini comme c 2.2),

c 3.2) représente $X_2$–S',

c 3.3) est défini comme dans a 3.1), n=3,

c 3.4) est défini comme en a 3.2) ou a 3.3), ou ses sels physiologiquement acceptables, caractérisé en ce que

a) on condense une Des-octapeptide B-23–30-insuline de formule II

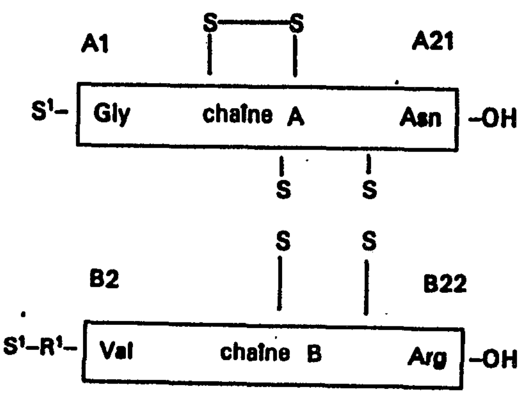


(II)

dans laquelle

$R^1$ désigne Phe ou une liaison et $S^1$ désigne un groupe protecteur du groupe amino pouvant être éliminé par solvolyse protonique ou par -élimination, comme le radical tert-butyloxycarbonyle

(Boc), tert-amyloxycarbonyle(Aoc) ou méthylsulfonyléthyloxycarbonyle (Msc) avec un peptide de formule III dans laquelle

$R^{30}$ ou $R^{31}$ on les significations ci-dessus, $S^2$ représente l'hydrogène, Bzl ou $Bu^t$ et $S^3$ représentent un groupe protecteur Boc, Moc, Fmoc ou Z, les groupes COOH–, OH–, SH–, $NH_2$–, guanidino et/ou imidazole libres présents dans les radicaux $R^{30}$ et $R^{31}$ étant, si nécessaire, protégés de façon connue en soi et on sépare de façon connue en soi les groupes protecteurs éventuellement présents, ou

b) on fait réagir une Des-B30-insuline de formule I, dans laquelle $R^1$ représente H ou H–Phe et l'extrémité C-terminal $R^{30}$–$R^{31}$ représente OH, en présence de trypsine ou d'une endopeptidase analogue à la trypsine avec un composé de formule IV dans laquelle $R^{30}$ et $R^{31}$ ont les significations définies dans les revendications 1 à 3, les fonctions COOH–, OH–, SH–, ω–$NH_2$–, guanidino-, et/ou imidazole libres présentes étant, si nécessaire, protégées de façon connue en soi et ensuite, on sépare de façon connue en soi les groupes protecteurs éventuellement présents et on transforme éventuellement les composés obtenus selon a) ou b) en leurs sels physiologiquement acceptables.

4. Procédé selon l'une des revendications 1 à 3, caractérisé en ce que l'on prépare un dérivé de l'insuline de formule I dans laquelle $R^1$ représente H–Phe.

5. Procédé selon l'une ou plusieurs des revendications 1 à 4, caractérisé en ce que l'on prépare un dérivé de l'insuline de formule I, dans laquelle $R^{30}$ représente Ala, Thr ou Ser.

6. Procédé selon une ou plusieurs des revendications 1 à 5, caractérisé en ce que l'on prépare un dérivé de l'insuline de formule I, dans laquelle la chaîne A et la chaîne (B2–29) présente la séquence de l'insuline humaine.

7. Procédé selon une ou plusieurs des revendications 1 à 6, caractérisé en ce que l'on prépare un dérivé de l'insuline de formule I dans laquelle les restes d'aminoacide X, $X^N$ et $X^B$ ainsi que les restes Y et Z sont sous la configuration L.

8. Procédé de préparation d'un dérivé de l'insuline de formule I dans laquelle les radicaux d'aminoacide X, $X^N$, $X^B$, Y et Z sont définis comme dans la revendication 7, caractérisé en ce que l'on coupe par voie chimique et/ou enzymatique une proinsuline, un dérivé de proinsuline, un dérivé de pré-proinsuline ou un intermédiaire de ses composés et on les transforment éventuellement en leurs sels physiologiquement acceptables.

9. Procédé de préparation selon l'une ou plusieurs des revendications 2 à 8, caractérisé en ce que l'on prépare un dérivé del'insuline de formule I, dans laquelle S ou S' représentent ($C_1$ à $C_6$)-alkoxy, alkylamino, ($C_3$ à $C_6$)-cycloalkoxy, $NH_2$, ($C_1$ à $C_6$)-alkylamino, di-($C_1$ à $C_6$)-alkylamino, amino-($C_2$ à $C_6$)-alkoxy, ($C_1$ à $C_4$)-alkylamino-($C_2$ à $C_6$)-alkoxy, di-($C_1$ à $C_4$)-alkylamino-($C_2$ à $C_6$)-alkoxy, tri-($C_1$ à $C_4$)-ammonio-($C_2$ à $C_6$)-alkoxy, amino-($C_2$ à $C_6$)-alkylamino, [($C_1$ à $C_4$)-alkylamino]-$C_2$ à $C_6$)-alkylamino, [di-($C_1$ à $C_4$)-alkylamino]-($C_2$ à $C_6$)-alkylamino ou [tri-($C_1$ à $C_4$)-alkylamino]-($C_2$ à $C_6$)-alkylamino et $S^B$ a l'une des 8 derniers significations citées.

10. Procédé selon une ou plusieurs des revendications 1 à 9, caractérisé en ce que l'on ajoute encore au dérivé de l'insuline un agent d'isotonicité approprié et un agent de conservation approprié, le dérivé d'insuline de formule I se trouvant dissous et/ou en suspension et le produit présentant un pH compris entre 2,5 et 8,5.

11. Procédé selon la revendication 10, caractérisé en ce que l'on ajoute encore un tampon approprié de façon que le pH soit finalement compris entre 5,0 et 8,5.

12. Procédé selon la revendication 10 ou 11, caractérisé en ce que l'on provoque encore un effet retard supplémentaire par addition de zinc en quantité telle que le produit contienne jusqu'à 100 microgrammes de zinc par 100 UI.

13. Procédé selon une ou plusieurs des revendications 10 à 12, caractérisé en ce que le dérivé d'insuline de formule I est sous la forme d'un sel alcalin ou du sel d'ammonium.

14. Procédé selon une ou plusieurs des revendications 10 à 13, caractérisé en ce qu'une proportion quelconque d'un ou de plusieurs dérivés de l'insuline de formule I ou un dérivé de l'insuline de formule I est en mélange avec d'autres de ces dérivés de l'insuline, chacun indépendamment l'un de l'autre, sous forme dissoute, amorphe et/ou cristalline.

15. Procédé selon une ou plusieurs des revendications 10 à 14, caractérisé en ce qu'on incorpore une quantité appropriée d'un adjuvant à effet retard.

16. Procédé selon la revendication 15, caractérisé en ce que ce principe retardant est employé en combinaison avec la totalité de la substance active ou avec des parties de celle-ci ou avec un ou plusieurs dérivés de l'insuline de formule I en mélange.

17. Procédé selon la revendication 15 ou 16, caractérisé en ce que l'on incorpore une combinaison de plusieurs adjuvants différents à effet retard.

18. Utilisation d'un dérivé de l'insuline de formule I selon la revendication 1, dans laquelle,

$R^1$ signifie H ou H–Phe,

$R^{30}$ représente le radical d'un L-aminoacide neutre codable génétiquement et

$R^{31}$ représente un groupe organique physiologiquement inoffensif, de caractère basique, ayant jusqu'à 50 atomes de C, à la structure duquel participent 0 à 3 α-aminoacides et dont la fonction carboxy terminal éventuellement présente peut se trouver sous forme libre, sous forme de fonction ester, de fonction amine, sous forme de lactone ou bien réduite en $CH_2OH$–, $R^1$ représentant H-Phe et l'extrémité C-terminal $R^{30}$–$R^{31}$ étant α)-Thr$(Arg)_m$–OH, -Ala-$(Arg)_m$–OH ou -Ser-$(Arg)_m$–OH avec m=1 ou 2 ou β) $R^{30}$ étant Thr et $R^{31}$ étant a) un aminoacide, b) un peptide ou c) un amide ou un ester de a) ou b), de préférence cependant de l'insuline-B31-Arg–OH ou l'insuline-B31-Arg-Arg–OH, d'un ester ou d'un amide

ou d'un sel physiologiquement acceptable de ceux-ci pour la préparation d'un médicament contre le diabète sucré.

**Claims for the Contracting States BE, CH, DE, FR, GB, IT, LI, LU, NL, SE**

1. An insulin derivative of the formula I

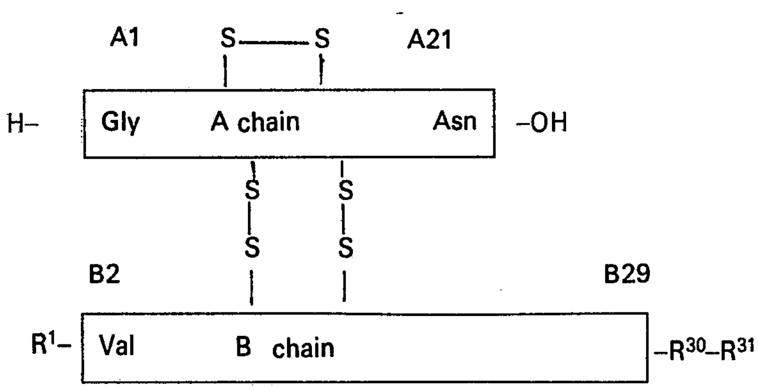

(I)

in which

$R^1$ denotes H or H–Phe,

$R^{30}$ represents the radical of a neutral L-aminoacid which can be genetically coded and

$R^{31}$ represents a physiologically acceptable organic group of basic character having up to 50 carbon atoms, in the build-up of which 0 to 3 α-aminoacids participate and in which the optional terminal carboxyl group can be present in the free form, as an ester function, as an amide function, as a lactone or reduced to $CH_2OH$, and wherein, if $R^1$ represents H-Phe, the C-terminus $-R^{30}-R^{31}$ cannot denote -Thr-$(Arg)_m$–OH, -Ala-$(Arg)_m$–OH or -Ser-$(Arg)_m$–OH, where m is 1 or 2, with the provision that also compounds are excluded in which simultaneous by $R^1$ denotes H–Phe, $R^{30}$ denotes Thr and $R^{31}$ denotes a) an amino acid, b) a peptide or c) an amide or ester of a) or b), characterized by an isoelectric point between 5.8 and 8.5, or physiologically acceptable salts thereof.

2. An insulin derivative of the formula I as claimed in claim 1, characterized in

$R^{31}$ representing a radical of the formula $-X_nS$, in which

n is 0, 1, 2 or 3,

X representing identical or different radicals of naturally ocurring neutral or basic L-aminoacids and/or of the D-aminoacids corresponding to these, and

S denoting OH or a physiologically acceptable group which blocks the carboxyl group and which, if n is 0, carries a positively charged or protonatable basic radical, or if $n > 0$, can carry such a radical, and in which the C-terminus –X–S can also represent the radical of an amino-acid reduced to the corresponding alcohol or, if n is 2 or 3, can represent the homoserine-lactone radical.

3. An insulin derivative of the formula I, in which $R^{30}$ represents the radical of a neutral L-aminoacid which can be genetically coded

a) $R^1$ denotes H and

$R^{31}$ a 1) denotes a physiologically acceptable group $S^B$ which blocks the carboxyl group and carries a positively charged or protonatable basic radical,

a 2.1) represents $X^N–S^B$, in which $X^N$ denotes the radical of a naturally occurring neutral L-aminoacid or the D-form thereof,

a 2.2) represents $X^B$–S, in which $X^B$ denotes the radical of a naturally occurring basic L-aminoacid or the D-form thereof and S denotes OH or a group which blocks the carboxyl group and optionally carries a positively charged or protonable basic radical,

a 2.3) represents the radical Y of a basic aminoacid $X^B$ reduced to the corresponding alcohol,

a 3.1) represents $-X_n$–S, in which n is 2 or 3, X denotes the radicals $X^N$ and/or $X^B$ and, if all the radicals X are $X^N$, S can denote only $S^B$,

a 3.2) represents $-X_n$–Y, in which n is 1 or 2,

a 3.3) represents $-X^B$–Z, $-X^B–X^N$–Z, $-X^N–X^B$–Z or $-X^B–X^B$–Z, in which z is Y or denotes the homoserine-lactone radical, or

b) $R^1$ denotes H–Phe and $R^{30}$ denotes Thr,

$R^{31}$ b 1) is as defined under a 1),

b 2) is as defined under a 2.3),

b 3) is as defined under a 3.2)or 3.3), or

c) $R^1$ denotes H–Phe and $R^{30}$ denotes the radical of another neutral L-aminoacid which can be genetically coded other than Thr,

$R^{31}$ c 1) is as defined under a 1),

c 2.1) is as defined under a 2.1)

c 2.2) denotes Lys–OH, D-Lys–OH, D-Arg–OH, Hyl–OH, D-Hyl–OH, Orn–OH, D-Orn–OH, Cit–OH, D-Cit–OH, His–OH or D-His–OH,

c 2.3) represents $X^B$–S', in which S' has the meaning of S, with the exception of OH,

c 2.4) is as defined under a 2.3),

c 3.1) represents X–X'–OH or –X'–X–OH, in which X' is as defined unter c 2.2),

c 3.2) represents $X_2$–S',

c 3.3) is as defined under a 3.1), in which n is 3,

c 3.4) is as defined under a 3.2) or a 3.3), or pysiologically acceptable salts thereof.

4. An insulin derivative as claimed in any one of claims 1 to 3, characterized in $R^1$ representing H–Phe.

5. An insulin derivative as claimed in any one of claims 1 to 4, characterized in $R^{30}$ representing Ala, Thr or Ser.

6. An insulin derivative as claimed in any one of claims 1 to 5, characterized in the A chain and the (B2–29) chain having the sequence of human insulin.

7. An insulin derivative as claimed in any one of claims 1 to 6, characterized in the aminoacid radicals X, $X^N$ and $X^B$ and the radicals Y and Z being in the L-configuration.

8. An insulin derivative as claimed in any one of claims 2 to 7, characterized in S or S′ representing ($C_1$ to $C_6$)-alkoxy, ($C_3$–$C_6$)-cycloalkoxy, $NH_2$, ($C_1$ to $C_6$)-alkylamino, di-($C_1$ to $C_6$)-alkylamino, amino-($C_2$ to $C_6$)-alkoxy, ($C_1$ to $C_4$)-alkylamino-($C_2$ to $C_6$)-alkoxy, di-($C_1$ to $C_4$)-alkylamino-($C_2$ to $C_6$)-alkoxy, tri-($C_1$ to $C_4$)-ammonio-($C_2$ to $C_6$)-alkoxy, amino-($C_2$ to $C_6$)-alkylamino, [($C_1$ to $C_4$)-alkylamino]-($C_2$ to $C_6$)-alkylamino, [di-($C_1$ to $C_4$)-alkylamino]-($C_2$ to $C_6$)-alkylamino [di-($C_1$ to $C_4$)-alkylamino]-$C_2$ to $C_6$)-alkylamino or [tri-($C_1$ to $C_4$)-alkylamino]-($C_2$ to $C_6$)-alkylamino and $S^B$ having one of the last eight meanings mentioned.

9. A process for the preparation of an insulin derivative of the formula I as claimed in any one of claims 1 to 8, characterized in

a) condensing a des-octapeptide (B23–30)-insulin of the formula II

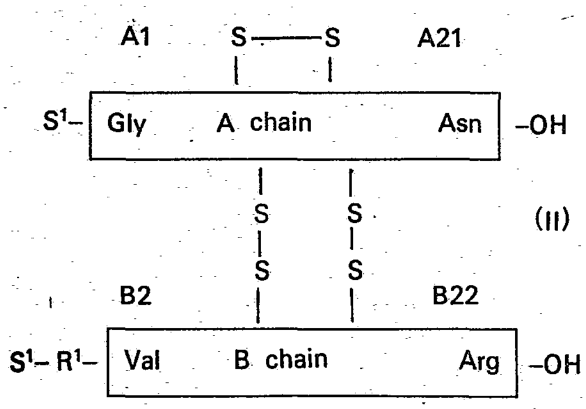

(II)

in which $R^1$ denotes Phe or a bond and $S^1$ denotes an amino-protective group which can be split off by proton solvolysis or by β-elimination, such as the tert.butoxycarbonyl (Boc), the tert.-amyloxycarbonyl (Aoc) or the methylsulfonylethoxycarbonyl (Msc) radical, with a peptide of the formula III

H-Gly-Phe-Phe-Tyr($S^2$)-Thr($S^2$)-Pro-Lys($S^3$)-$R^{30}$–$R^{31}$ (III)

in which $R^{30}$ and $R^{31}$ have the meanings defined in claims 1 and 2, $S^2$ represents hydrogen, Bzl or $Bu^t$ and $S^3$ represent a urethane-protective group, such as Boc, Moc, Fmoc or Z, it being possible, if necessary, for free COOH, OH, SH, $NH_2$ guanidino and/or imidazole groups present in the radicals $R^{30}$ and $R^{31}$ to be protected in a manner which is known per se, and, if appropriate, splitting off the protective groups present in a manner which is known per se,

b) reacting, in the presence of trypsin or a trypsinlike endopeptidase, a des-B30-insulin of the formula I in which $R^1$ represents H or H–Phe and the C-terminus $R^{30}$–$R^{31}$ together represents OH, with a compound of the formula IV

H–$R^{30}$–$R^{31}$ (IV)

in which $R^{30}$ and $R^{31}$ have the meanings defined in claims 1 to 3 and free COOH, OH, SH, ω–$NH_2$, guanidino and/or imidazole functions present are, if necessary, protected in a manner which is known per se, and then, if appropriate, splitting off the protective groups present in a manner which is known per se, or

c) for the preparation of an insulin derivative as claimed in claim 7, chemically and/or enzymatically splitting a proinsulin, proinsulin derivative or preproinsulin derivative or an intermediate of these compounds, and, if appropriate, converting a compound obtained according to a)–c) into its physiologically acceptable salt.

10. An insulin derivative of the formula I

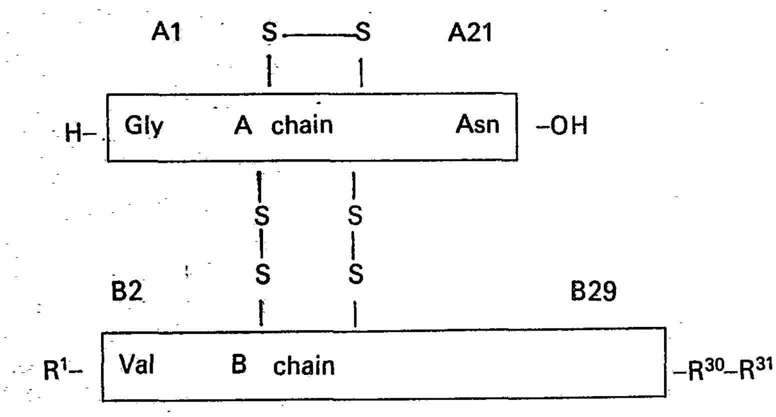

(I)

in which

$R^1$ denotes H or H–Phe,

$R^{30}$ represents the radical of a neutral L-aminoacid which can be genetically coded and

$R^{31}$ represents a physiologically acceptable organic group of basic character having up to 50 carbon atoms, in the build-up of which 0 to 3 α-aminoacids participate and in which the optional

terminal carboxyl group can be present in the free form, as an ester function, as an amide function, as a lactone or reduced to $CH_2OH$, and wherein, if $R^1$ represents H–Phe, the C-terminus $-R^{30}-R^{31}$ cannot denote -Thr-$(Arg)_m$–OH, -Ala-$(Arg)_m$–OH or -Ser-$(Arg)_m$–OH, where m is 1 or 2, characterized by an isoelectric point between 5.8 and 8.5, or physiologically acceptable salts thereof or, respectively, such a derivative according to one or more of claims 1 to 8 for use as medicine, particularly for the treatment of diabetes mellitus.

11. A medicament containing an insulin derivative as claimed in claim 10.

12. A medicament consisting of a pharmaceutically acceptable excipient and an active compound, characterized in containing, as the active compound, an insulin derivative with an isoelectric point between 5.8 and 8.5, of the formula I, in which

$R^1$ denotes H or H–Phe,

$R^{30}$ represents the radical of a neutral L-aminoacid which can be genetically coded and

$R^{31}$ represents a physiologically acceptable organic group of basic character with up to 50 carbon atoms, in the build-up of which 0 to 3 α-aminoacids participate and in which the optional terminal carboxyl group present can be in the free form, as an ester function, as an amide function, as a lactone or reduced to $CH_2OH$, or one of its physiologically acceptable salts.

13. An agent as claimed in claim 12, characterized in containing as the active compound insulin-B31-Arg–OH or insulin-B31-Arg-Arg–OH, esters or amides or the physiologically tolerable salts thereof.

14. An agent as claimed in either of claims 12 and 13, characterized in having a pH value between 2.5 and 8.5 and containing a suitable isotonicity agent and a suitable preservative and in which the insulin derivative of the formula I being in dissolved form and/or in suspension.

15. An agent as claimed in one or more of claims 12 to 14, characterized in containing suitable buffer and having a Ph value between 5.0 and 8.5.

16. An agent as claimed in one or more of claims 12 to 15, characterized in continaing between 0 and 100 µg of zinc/100 I.U.

17. An agent as claimed in one or more of claims 12 to 16, characterized in the peptide of the formula I being in the form of an alkali metal salt or the ammonium salt.

18. An agent as claimed in one or more of claims 12 to 17, characterized in any desired amount of one or more insulin derivatives of the formula I or an insulin derivative of the formula I being mixed with other insulin derivatives of this type in dissolved, amorphous and/or crystalline form in each case, independently of one another.

19. An agent as claimed in one or more of claims 12 to 18, characterized in containing a suitable amount of an auxiliary having a delaying action.

20. An agent as claimed in claim 19, characterized in this delayed action principle being used in combination with the entire content of active compound or with parts thereof or one or more insulin derivatives of the formula I, in a mixture.

21. An agent as claimed in one or more of claims 12 to 20, characterized in containing various insulin derivatives of the formula I in combination with several different auxiliaries having a delaying action.

22. The process for the preparation of an agent as claimed in either of claims 11 and 12, characterized in bringing an insulin derivative of the formula I into a suitable form for administration.

**Claims for the Contracting State AT**

1. A process for the production of an insulin derivative of the formula I

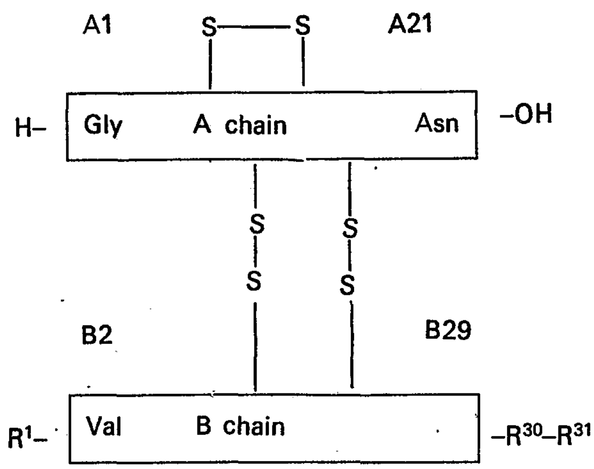

in which

$R^1$ denotes H or H–Phe,

$R^{30}$ represents the radical of a neutral L-aminoacid which can be genetically coded and

$R^{31}$ represents a physiologically acceptable organic group of basic character having up to 50 carbon atoms, in the build-up of which 0 to 3 α-aminoacids participate and in which the optional

terminal carboxyl group can be present in the free form, as an ester function, as an amide function, as a lactone or reduced to $CH_2OH$, and wherein, if $R^1$ represents H-Phe, the C-terminus -$R^{30}$–$R^{31}$ cannot denotes -Thr-$(Arg)_m$–OH, -Ala-$(Arg)_m$–OH or -Ser-$(Arg)_m$–OH, where m is 1 or 2, with the provision that, also compounds are excluded in which simultaneous by $R^1$ denotes H–Phe, $R^{30}$ denotes Thr and $R^{31}$ denotes a) an amino acid, b) a peptide or c) an amide or ester of a )or b), having an isoelectric point between 5.8 and 8.5, or physiologically acceptable salts thereof, characterized in

a) condensing a des-octapeptide (B23–30)-insulin of the formula II

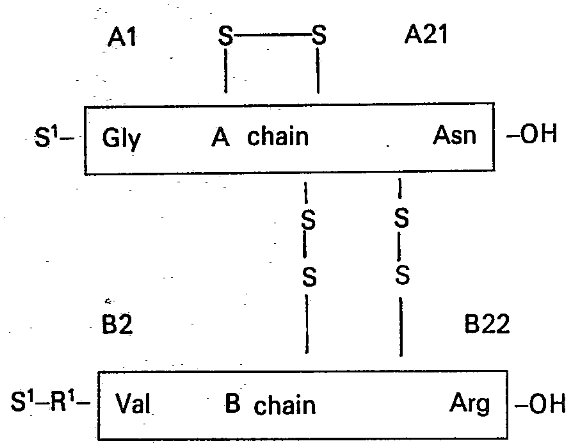

(II)

in which $R^1$ denotes Phe or a bond and $S^1$ denotes an amino-protective group which can be split off by proton solvolysis or by β-elimination, such as the tert.butoxycarbonyl (Boc), the tert.-amyloxycarbonyl (Aoc) or the methylsulfonylethoxycarbonyl (Msc) radical, with a peptide of the formula III

H-Gly-Phe-Phe-Tyr($S^2$)-Thr($S^2$)-Pro-Lys($S^3$)-$R^{30}$–$R^{31}$ (III)

in which $R^{30}$ and $R^{31}$ have the meanings defined in claims 1 and 2, $S^2$ represents hydrogen, Bzl or $Bu^t$ and $S^3$ represent a urethane-protective group, such as Boc, Moc, Fmoc or Z, it being possible, if necessary, for free COOH, OH, SH, $NH_2$ guanidino and/or imidazole groups present in the radicals $R^{30}$ and $R^{31}$ to be protected in a manner which is known per se, and, if appropriate, splitting off the protective groups present in a manner which is known per se,

b) reacting, in the presence of trypsin or a trypsin-like endopeptidase, a des-B30-insulin of the formula I in which $R^1$ represents H or H–Phe and the C-terminus $R^{30}$–$R^{31}$ together represents OH, with a compound of the formula IV

H–$R^{30}$–$R^{31}$ (IV)

in which $R^{30}$ and $R^{31}$ have the meanings defined in claims 1 to 3 and free COOH, OH, SH, ω–$NH_2$, guanidino and/or imidazole functions present are, if necessary, protected in a manner which is known per se, and then, if appropriate, splitting off the protective groups present in a manner which is known per se, and, optionally transforming the compounds obtained according to a) or b) into their physiologically tolerable salts.

2. A process as claimed in claim 1, characterized in producing an insulin derivative of the formula I as claimed in claim 1, in which

$R^{31}$ represents a radical of the formula $-X_nS$, in which

n is 0, 1, 2 or 3,

X represents identical or different radicals of naturally ocurring neutral or basic L-aminoacids and/or of the D-aminoacids corresponding to these, and

S denotes OH or a physiologically acceptable group which blocks the carboxyl group and which, if n is 0, carries a positively charged or protonatable basic radical, or if n > 0, can carry such a radical, and in which the C-terminus –X–S can also represent the radical of an amino-acid reduced to the corresponding alcohol or, if n is 2 or 3, can represent the homoserine-lactone radical.

3. A process for the production of an insulin derivative of the formula I, in which $R^{30}$ represents the radical of a neutral L-aminoacid which can be genetically coded

a) $R^1$ denotes H and

$R^{31}$ a 1) denotes a physiologically acceptable group $S^B$ which blocks the carboxyl group and carries a positively charged or protonatable basic radical,

a 2.1) represents $X^N$–$S^B$, in which $X^N$ denotes the radical of a naturally occurring neutral L-aminoacid or the D-form thereof,

a 2.2) represents $X^B$–S, in which $X^B$ denotes the radical of a naturally occurring basic L-aminoacid or the D-form thereof and S denotes OH or a group which blocks the carboxyl group and optionally carries a positively charged or protonatable basic radical,

a 2.3) represents the radical Y of a basic aminoacid $X^B$ reduced to the corresponding alcohol,

a 3.1) represents $-X_n-S$, in which n is 2 or 3, X denotes the radicals $X^N$ and/or $X^B$ and, if all the radicals X are $X^N$, S can denote only $S^B$,

a 3.2) represents $-X_n-Y$, in which n is 1 or 2,

a 3.3) represents $-X^B-Z$, $-X^B-X^N-Z$, $-X^N-X^B-Z$ or $-X^B-X^B-Z$, in which z is Y or denotes the homoserine-lactone radical, or

b) $R^1$ denotes H–Phe and $R^{30}$ denotes Thr,

$R^{31}$ b 1) is as defined under a 1),

b 2) is as defined under a 2.3),

b 3) is as defined under a 3.2) or 3.3), or

c) $R^1$ denotes H-Phe and $R^{30}$ denotes the radical of another neutral L-aminoacid which can be genetically coded, other than Thr,

$R^{31}$ c 1) is as defined under a 1),

c 2.1) is as defined under a 2.1),

c 2.2) denotes Lys–OH, D-Lys–OH, D-Arg–OH, Hyl–OH, D-Hyl–OH, Orn–OH, D-Orn–OH, Cit–OH, D-Cit–OH, His–OH or D-His–OH,

c 2.3) represents $X^B-S'$, in which S' has the meaning of S, with the exception of OH,

c 2.4) is as defined under a 2.3),

c 3.1) represents X–X'–OH or –X'–X–OH, in which X' is as defined under c 2.2),

c 3.2) represents $X_2-S'$,

c 3.3) is as defined under a 3.1), in which n is 3,

c 3.4) is as defined under a 3.2) or a 3.3), or pysiologically acceptable salts thereof, characterized in

a) condensing a des-octapeptide (B23–30)-insulin of the formula II

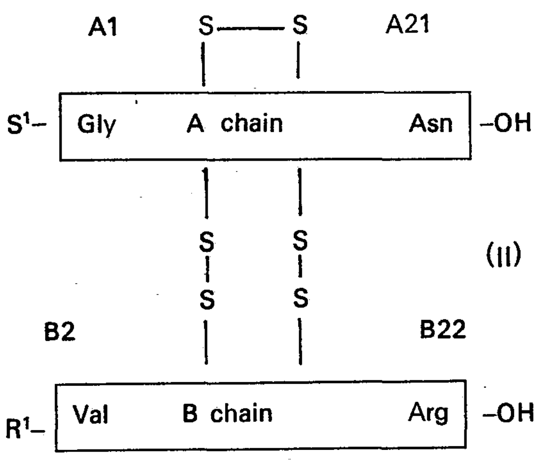

(II)

in which $R^1$ denotes Phe or a bond and $S^1$ denotes an amino-protective group which can be split off by proton solvolysis or by β-elimination, such as the tert.butoxycarbonyl (Boc), the tert.-amyloxycarbonyl (Aoc) or the methylsulfonylethoxycarbonyl (Msc) radical, with a peptide of the formula III in which $R^{30}$ and $R^{31}$ have the meanings defined above, $S^2$ represents hydrogen, Bzl or $Bu^t$ and $S^3$ represent a urethan-protective group, such as Boc, Moc, Fmoc or Z, it being possible, if necessary, for free COOH, OH, SH, $NH_2$ guanidino and/or imidazole groups present in the radicals $R^{30}$ and $R^{31}$ to be protected in a manner which is known per se, and, if appropriate, splitting off the protective groups present in a manner which is known per se,

b) reacting, in the presence of trypsin or a trypsin-like endopeptidase, a des-B30-insulin of the formula I in which $R^1$ represents H or H–Phe and the C-terminus $R^{30}-R^{31}$ together represents OH, with a compound of the formula IV in which $R^{30}$ and $R^{31}$ have the meanings defined in claims 1 to 3 and free COOH, CH, SH, $\omega-NH_2$, guanidino and/or imidazole functions present are, if necessary, protected in a manner which is known per se, and then, if appropriate, splitting off the protective groups present in a manner which is known per se, and optionally transforming the compounds obtained according to a) or b) into their physiologically tolerable salts.

4. A process as claimed in one or more of claims 1 to 3, characterized in producing an insulin derivative of formula I in which $R^1$ represents H–Phe.

5. A process as claimed in one or more of claims 1 to 4, characterized in producing an insulin derivative of formula I, in which $R^{30}$ represents Ala, Thr or Ser.

6. A process as claimed in one or more of claims 1 to 5, characterized in producing an insulin derivative of formula I, in which the A chain and the (B2–29) chain have the sequence of human insulin.

7. A process as claimed in one or more of claims 1 to 6, characterized in producing an insulin derivative of formula I, in which the aminoacid radicals X, $X^N$ and $X^B$ and the radicals Y and Z being in the L-configuration.

8. A process for the production of an insulin derivative of formula I in which the amino acid radicals X, $X^N$, $X^B$, Y and Z are defined as in claim 7, characterized in splitting a proinsulin, proinsulin derivative or preproinsulin derivative or an intermediate of these compounds, and, if appropriate, converting a compound obtained according to a)–c) into its physiologically acceptable salt.

9. A process as claimed in any one of claims 2 to 8, characterized in producing an insulin derivate of formula I, in which S or S' represents ($C_1$ to $C_6$)-alkoxy, ($C_3$–$C_6$)-cycloalkoxy, $NH_2$, ($C_1$ to $C_6$)-alkylamino, di-($C_1$ to $C_6$)-alkylamino, amino-($C_2$ to $C_6$)-alkoxy, ($C_1$ to $C_4$)-alkylamino-($C_2$ to $C_6$)-alkoxy, di-($C_1$ to $C_4$)-alkylamino-($C_2$ to $C_6$)-alkoxy, tri-($C_1$ to $C_4$)-ammonio-($C_2$ to $C_6$)-alkoxy, amino-($C_2$ to $C_6$)-alkylamino, [($C_1$ to $C_4$)-alkylamino]-($C_2$ to $C_6$)-alkylamino, [di-($C_1$ to $C_4$)-alkylamino]-($C_2$ to $C_6$)-alkylamino or [tri-($C_1$ to $C_4$)-alkylammonio]-($C_2$ to $C_6$)-alkylamino and $S^B$ has one of the last eight meanings mentioned.

10. A process as claimed in one or more of claims 1 to 9, characterized in adding to the insulin derivative a suitable isotonicity agent and a suitable preservative, wherein the insulin derivative of the formula I being in dissolved form and/or in suspension and the product having a pH-value between 2.5 and 8.5.

11. A process as claimed in claim 10, characterized in adding a suitable buffer in a manner that at least there is a pH value between 5.0 and 8.5.

12. A process as claimed in claims 10 or 11, characterized in creating an additional depot action by addition of such a amount of zinc that product contains up to 100 µg of zinc/100 I.U.

13. A process as claimed in one or more of claims 10 to 12, characterized in the peptide of the formula I being in the form of an alkali metal salt or the ammonium salt.

14. A process as claimed in one or more of claims 10 to 13, characterized in that any desired a mount of one or more insulin derivatives of the formula I or an insulin derivative of the formula I in a mixture with other insulin derivatives of this type is present in dissolved, amorphous and/or crystalline form in each case, independently of one another.

15. A process as claimed in one or more of claims 10 to 14, characterized in that a suitable amount of an auxiliary having a delaying action is incorporated.

16. A process as claimed in claim 15, characterized in that this delayed action principle being used in combination with the entire content of active compound or with parts thereof or one or more insulin derivatives of the formula I, in a mixture.

17. A process as claimed in claims 15 or 16, characterized in that various insulin derivatives of the formula I in combination with several different auxiliaires having a delaying action are incorporated.

18. The use of an insulin derivatives as claimed in claim 1 in which

$R^1$ denotes H or H–Phe,

$R^{30}$ represents the radical of a neutral L-aminoacid which can be genetically coded and

$R^{31}$ represents a physiologically acceptable organic group of basic character having up to 50 carbon atoms, in the build-up of which 0 to 3 α-aminoacids participate and in which the optional terminal carboxyl group can be present in the free form, as an ester function, as an amide function, as a lactone or reduced to $CH_2OH$, wherein $R^1$ denotes H-Phe and the C-terminus -$R^{30}$–$R^{31}$ is α) -Thr-$(Arg)_m$–OH, -Ala-$(Arg)_m$OH or -Ser-$(Arg)_m$–OH, with m=1 or 2, or β) $R^{30}$ is Thr and $R^{31}$ is a) an aminoacid, b) a peptide or c) an amide or ester of a) or b), preferably, however, of insulin-B31-Arg–OH or insulin-B31-Arg-Arg–OH, of an ester or amide or a physiologically tolerable salt thereof for the production of medicaments against diabetes mellitus.